# EUROPEAN PATENT APPLICATION

(11) **EP 4 549 426 A1**
(43) Date of publication of application: **07.05.2025**
(21) Application number: 23831479.3
(22) Date of filing: 28.06.2023
(51) Int. Cl.: C07C 51/347, C07C 57/03, C07D 309/04, C07B 61/00, C07C 67/30, C07C 69/587

(54) **METHOD FOR PRODUCING VERY-LONG-CHAIN POLYUNSATURATED FATTY ACID**

(30) Priority: 28.06.2022 JP 2022103927
(71) Applicant: Nissui Corporation, Tokyo 105-8676 (JP)
(72) Inventor: TANAKA, Kumpei, Hachioji-shi, Tokyo 192-0991 (JP); OHTSUKA, Naomi, Hachioji-shi, Tokyo 192-0991 (JP); SATO, Seizo, Hachioji-shi, Tokyo 192-0991 (JP)
(74) Representative: Mewburn Ellis LLP
(86) International application number: PCT/JP2023/023884
(87) International publication number: WO 2024/005039

(57) **Abstract**

The present invention provides a method for producing a very-long-chain polyunsaturated fatty acid, the method comprising reacting a compound represented by Formula II: R¹-MgX¹ with Formula III: X²-(CH₂)ₘ-X in a solvent to obtain a compound represented by Formula I: R¹-(CH₂)ₘ-X.

## Description

### Technical Field

The present invention relates to a method for producing a very-long-chain polyunsaturated fatty acid. The present invention also relates to a method for producing a very-long-chain polyunsaturated fatty acid used for pharmaceutical applications. In addition, the present invention relates to a method for producing a very-long-chain polyunsaturated fatty acid using an environmentally benign reaction.

### Background Art

It is known that natural resources contain long-chain polyunsaturated fatty acids (LC-PUFA) such as eicosapentaenoic acid (EPA) which is an n-3 type polyunsaturated fatty acid and arachidonic acid (ARA) which is an n-6 type polyunsaturated fatty acid. It is also known that a very-long-chain polyunsaturated fatty acid (VLC-PUFA, e.g., n-3 VLC-PUFA) having a longer carbon chain than LC-PUFA such as EPA is also contained, and the concentration thereof is trace as compared with LC-PUFA. Recently, physiological activities of VLC-PUFA and their hydroxylated derivatives known as elobanoid have been reported (Patent Documents 1 and 2, Non-Patent Document 1). Polyunsaturated fatty acids (PUFA) such as LC-PUFA and VLC-PUFA are known to be easily oxidized and easily isomerized because they abundantly have a double bond or active methylene sandwiched between double bonds in their structures. Therefore, for the chemical reaction of the PUFA, it is necessary to take care to control the side reaction as described above or to apply a reaction in which the side reaction does not occur.

As synthesis examples of VLC-PUFA, a method of synthesizing C28: 6n-3 from docosahexaenoic acid (DHA: C22: 6n-3) (Patent Document 3) and a method of synthesizing C34: 3n-3 methyl ester from a higher alcohol which is a reductant of α-linolenic acid (ALA) (Non-Patent Document 2) are known. In the above methods, the numbers of reaction steps are large until VLC-PUFAs are synthesized from LC-PUFAs, and the yields are low. Under such circumstances, there has been a strong demand for the development of a method capable of synthesizing VLC-PUFAs quickly and in large quantities.

### Citation List

### Patent Documents

Patent Document 1: WO 2016/130522 A1
Patent Document 2: WO 2020/206448 A1
Patent Document 3: WO 2011/053892 A1

Non-Patent Document 1: Bazan, NG, Molecular Aspects of Medicine 64 (2018) 18-33
Non-Patent Document 2: Kling, MR et al., J. Chem. Parkin Trans. 1993, 1183-1189

### Summary of Invention

### Technical Problem

Even if the prior art is applied, it has not been possible to synthesize very-long-chain polyunsaturated fatty acids from a long-chain polyunsaturated fatty acid quickly and in large quantities, which is not a suitable method for production on an industrial scale. An object of the present invention is to provide a novel method for producing a very-long-chain polyunsaturated fatty acid.

### Solution to Problem

The present inventors have invented a method for producing VLC-PUFA by preparing a Grignard reagent from LC-PUFA. The above method also has improved the yield and has been a more suitable production method than the prior art methods for producing VLC-PUFA quickly and in large quantities.

The present specification includes the following disclosure of the invention.
[1] A method for producing a compound represented by Formula I or a salt thereof:

   R¹-(CH₂)ₘ-X (I),

   where R¹ is a C₁₈₋₃₀ hydrocarbon group containing from 3 to 7 double bonds;
   m is an integer selected from 1 to 19;
   X is CO₂H, CO₂R^{a}, or CH₂OR^{b};
   R^{a} is a C₁₋₆ alkyl; and
   R^{b} is an ether-forming group, a silyl ether-forming group, or an ester-forming group.
[2] The method according to [1], comprising obtaining the compound represented by Formula I by reacting, in a solvent, a compound represented by Formula II:

   R¹-MgX¹ (II)

   where X¹ is a halogen atom,
   with a compound represented by Formula III:

      X²-(CH₂)ₘ-X (III)
   where X² is selected from a halogen atom and a sulfonyloxy group.
[3] The method according to [2], wherein the reaction is carried out in the presence of a metal catalyst.
[4] The method according to [2] or [3], wherein the reaction is carried out in the presence of an additive.
[5] The method according to any of [1] to [4], wherein R¹ is a C₁₈, C₂₀ or C₂₂ hydrocarbon group containing from 3 to 6 double bonds.
[6] The method according to any of [1] to [5], wherein R¹ is a group represented by Formula XI:

   CH₃-(CH₂-CH=CH)ₐ-(CH₂)_{b}- (XI)

   where a is 5 and b is an integer selected from 2 to 14, or a is 6 and b is an integer selected from 1 to 11; or
   a group represented by Formula XII:

      CH₃-(CH₂)₃-(CH₂-CH=CH)_{c}-(CH₂)_{d}- (XII)
   where c is 3 and d is an integer selected from 5 to 17, or c is 4 and d is an integer selected from 2 to 14.
[7] The method according to [6], wherein R¹ is the group represented by Formula XI, in which a is 5 and b is 4, or a is 6 and b is 3, or
   R¹ is the group represented by Formula XII, in which c is 3 and d is 7, or c is 4 and d is 4.
[8] The method according to any of [1] to [7], wherein all double bonds contained in R¹ are of a cis type.
[9] The method according to any of [1] to [8], wherein m is 7, 9, 11, or 13.
[10] The method according to any of [3] to [9], wherein the metal catalyst is selected from a copper catalyst, a nickel catalyst, a palladium catalyst, and an iron catalyst.
[11] The method according to any of [3] to [10], wherein the metal catalyst is selected from the group consisting of Li₂CuCl₄, CuI, CuBr, CuCl, CuBr₂, CuCl₂, CuCN, CuCl₂·2LiCl, CuCN·2LiCl, CuBr/CH₃SCH₃, Cu(acac)₂, Cu(OAc)₂, NiCl₂, Ni(acac)₂, Pd(OAc)₂, Pd(PPh₃)₄, Pd(acac)₂, PdCl₂, Fe(acac)₃, and FeCl₃.
[12] The method according to any of [3] to [11], wherein the metal catalyst is Li₂CuCl₄.
[13] The method according to any of [4] to [12], wherein the additive is selected from a nitrogen-based additive, an unsaturated hydrocarbon-based additive, and a silyl-based additive, or a combination thereof.
[14] The method according to any of [4] to [13], wherein the additive is selected from NMP, DMPU, HMPA, HMTA, TMEDA, 1,3-butadiene, isoprene, 1-phenylpropyne and TMS-Cl, or a combination thereof.
[15] The method according to any of [3] to [14], wherein an amount of the metal catalyst is from 0.1 to 20 mol% with respect to the compound represented by Formula III.
[16] The method according to any of [2] to [15], wherein an amount of the compound represented by Formula II is from 1 to 5 equivalents with respect to the compound represented by Formula III.
[17] The method according to any of [4] to [16], wherein an amount of the additive is from 0.1 to 10 equivalents with respect to the compound represented by Formula III.
[18] The method according to any of [2] to [17], wherein X¹ is a bromine atom.
[19] The method according to any of [1] to [18], wherein CH₂OR^{b} represents an ether structure having from 2 to 20 carbon atoms, a silyl ether structure having from 4 to 20 carbon atoms, or an ester structure having from 2 to 20 carbon atoms.
[20] The method according to any of [1] to [19], wherein R^{b} is selected from t-butyl, benzyl, p-methoxyphenylbenzyl, trityl, methoxymethyl, methoxyethoxymethyl, tetrahydropyranyl, trimethylsilyl, triethylsilyl, t-butyldimethylsilyl, t-butyldiphenylsilyl, acetyl, pivaloyl, and benzoyl.
[21] The method according to any of [1] to [20], wherein X is CO₂H, and the compound represented by Formula I is a very-long-chain polyunsaturated fatty acid having from 20 to 50 carbon atoms and containing from 3 to 7 double bonds, or
   X is CO₂R^{a}, and the compound represented by Formula I is a C₁₋₆ alkyl ester of a very-long-chain polyunsaturated fatty acid having from 20 to 50 carbon atoms and containing from 3 to 7 double bonds.
[22] The method according to any of [1] to [21], wherein the compound represented by Formula I is a very-long-chain polyunsaturated fatty acid that is C₂₈₋₃₆: 5n-3, C₂₈₋₃₆: 4n-6, C₂₈₋₃₆: 6n-3, or C₂₈₋₃₆: 3n-6, or a C₁₋₆ alkyl ester thereof.
[23] The method according to any of [1] to [22], wherein the compound represented by Formula I is a very-long-chain polyunsaturated fatty acid that is C32: 4n-6, C34: 4n-6, C32: 5n-3, C34: 5n-3, C32: 6n-3, or C34: 6n-3, or a C₁₋₆ alkyl ester thereof.
[24] The method according to any of [1] to [23], wherein the compound represented by Formula I is a very-long-chain polyunsaturated fatty acid that is C32: 4n-6 or C34: 5n-3 or a C₁₋₆ alkyl ester thereof.
[25] The method according to any of [2] to [24], wherein the reaction comprises adding, in the solvent, a mixture containing the compound represented by Formula II:

   R¹-MgX¹ (II)

   to the compound represented by Formula III:

   X²-(CH₂)ₘ-X (III).
[26] The method according to any of [4] to [25], wherein the reaction is carried out in the solvent in the presence of the metal catalyst and the additive at a temperature of from -20°C to 35°C.
[27] The method according to any of [4] to [26], wherein the reaction comprises adding, in the solvent, a mixture containing the compound represented by Formula II:

   R¹-MgX¹ (II)

   to the compound represented by Formula III:

      X²-(CH₂)ₘ-X (III)
   in the presence of the metal catalyst and the additive over a period of time from 5 minutes to 80 minutes.
[28] The method according to any of [4] to [27], wherein the reaction comprises adding, in the solvent, a mixture containing the compound represented by Formula II:

   R¹-MgX¹ (II)

   to the compound represented by Formula III:

      X²-(CH₂)ₘ-X (III)
   in the presence of the metal catalyst and the additive at a rate of from 0.1 to 1000 mL/min.
[29] The method according to any of [2] to [28], further comprising converting, in the solvent, a compound represented by Formula IIc:

   R¹-X¹ (IIc)

   into the compound represented by Formula II:

   R¹-MgX¹ (II).
[30] The method according to [29], wherein the converting comprises adding, in the solvent, in the presence of magnesium, the compound represented by Formula IIc:

   R¹-X¹ (IIc)

   or a mixture containing the compound to convert the compound represented by Formula IIc into the compound represented by Formula II:

   R¹-MgX¹ (II).
[31] The method according to [30], wherein the converting comprises adding the compound represented by Formula IIc:

   R¹-X¹ (IIc)

   or a mixture containing the compound over 5 to 60 minutes.
[32] The method according to [30] or [31], wherein the converting comprises adding the compound represented by Formula IIc:

   R¹-X¹ (IIc)

   or a mixture containing the compound at a rate of from 0.1 to 1000 mL/min.
[33] The method according to any of [2] to [32], further comprising:
   converting a compound represented by Formula IIa:

      R¹-OH (IIa)
   into a compound represented by Formula IIb:

      R¹-OX³ (IIb)
   where X³ is a sulfonyl group;
   converting the compound represented by Formula IIb into the compound represented by Formula IIc:

      R¹-X¹ (IIc);

      and
   converting the compound represented by Formula IIc into the compound represented by Formula II:

      R¹-MgX¹ (II).
[34] The method according to any of [1] to [19] and [21] to [32], wherein X is CH₂OR^{b}, further comprising converting a compound represented by Formula Ia:

   R¹-(CH₂)ₘ-CH₂OR^{b} (Ia)

   into a compound represented by Formula V:

      R¹-(CH₂)ₘ-CH₂OH (V);

      and
   converting the compound represented by Formula V into a compound represented by Formula Ib:

      R¹-(CH₂)ₘ-CO₂H (Ib),
   wherein the compound represented by Formula Ib is a very-long-chain polyunsaturated fatty acid having from 20 to 50 carbon atoms and containing from 3 to 7 double bonds.
[35] The method according to any of [1] to [34], further comprising converting a very-long-chain polyunsaturated fatty acid into a C₁₋₆ alkyl ester.
[36] Use of a compound of Formula II for preparation of a very-long-chain polyunsaturated fatty acid or a C₁₋₆ alkyl ester thereof.
[37] Use of a compound of Formula III for preparation of a very-long-chain polyunsaturated fatty acid or a C₁₋₆ alkyl ester thereof.
[38] A method for producing a compound represented by Formula I or a salt thereof:

   R¹-(CH₂)ₘ-X (I),

   where R¹ is a C₁₈₋₃₀ hydrocarbon group containing from 3 to 7 double bonds,
   m is an integer selected from 1 to 19;
   X is CO₂H, CO₂R^{a}, or CH₂OR^{b};
   R^{a} is a C₁₋₆ alkyl; and
   CH₂OR^{b} is an ether structure, a silyl ether structure, or an ester structure,
   the method comprising:
      (1) converting a compound represented by Formula IV:

         R⁴-CO₂R² (IV),

         where R⁴ is a C₁₇₋₂₉ hydrocarbon group containing from 3 to 7 double bonds and R² is a hydrogen atom or an ester-forming group,
         into a compound represented by Formula IIa:

            R⁴-CH₂-OH (IIa);
      (2) converting the compound represented by Formula IIa into a compound represented by Formula IIb:

         R⁴-CH₂-OX³ (IIb)

         where X³ is a sulfonyl group;
      (3) converting the compound represented by Formula IIb into a compound represented by Formula IIc:

         R⁴-CH₂-X¹ (IIc)

         where X¹ is a halogen atom;
      (4) converting the compound represented by Formula IIc into a compound represented by Formula II:

         R⁴-CH₂-MgX¹ (II);

         and
      (5) reacting the compound represented by Formula II with a compound represented by Formula III to convert into the compound represented by Formula I:

         X²-(CH₂)ₘ-X (III),

         where X² is selected from a halogen atom and a sulfonyloxy group.
[39] The method according to [38], wherein (5) the reaction between the compound represented by Formula II and the compound represented by Formula III is performed in a solvent in the presence of a metal catalyst.
[40] The method according to [38], wherein (1) the conversion of the compound represented by Formula IV comprises reacting the compound represented by Formula IV with a reducing agent in a solvent;
   (2) the conversion of the compound represented by Formula IIa comprises reacting the compound represented by Formula IIa with a sulfonylating agent in a solvent in the presence of a base;
   (3) the conversion of the compound represented by Formula IIb comprises reacting the compound represented by Formula IIb with a halogenating agent in a solvent;
   (4) the conversion of the compound represented by Formula IIc comprises reacting the compound represented by Formula IIc with magnesium in a solvent; and
   (5) the reaction between the compound represented by Formula II and the compound represented by Formula III comprises reacting the compound represented by Formula II with the compound represented by Formula III in a solvent in the presence of a metal catalyst.
[41] The method according to any of [38] to [40], wherein (2) the conversion of the compound represented by Formula IIa and (3) the conversion of the compound represented by Formula IIb are performed by one-pot synthesis.
[42] The method according to any of [38] to [41], wherein the method comprises a one-pot synthesis for preparing the compound represented by Formula IIc in (3), the one-pot synthesis comprising:
   (2) reacting, in a solvent, the compound represented by Formula IIa with TsCl in the presence of one, or two or more amines to adjust the compound represented by Formula IIb:

      R⁴-CH₂-OX³ (IIb)

      where X³ is toluenesulfonyl, and
   (3) reacting the compound represented by Formula IIb with LiBr without isolation to prepare the compound represented by Formula IIc:

      R⁴-CH₂-X¹ (IIc)

      where X¹ is a bromine atom.
[43] The method according to any of [38] to [42], wherein (1) the conversion of the compound represented by Formula IV is performed by reacting, in a solvent, the compound represented by Formula IV with sodium bis(2-methoxyethoxy) aluminum hydride.
[44] A production method for obtaining a very-long-chain polyunsaturated fatty acid having from 20 to 50 carbon atoms and containing from 3 to 7 double bonds or a C₁₋₆ alkyl ester thereof by converting a long-chain polyunsaturated fatty acid having from 18 to 30 carbon atoms and containing from 3 to 7 double bonds or a C₁₋₆ alkyl ester thereof, the production method comprising, in the conversion step, reacting a Grignard reagent derived from the long-chain polyunsaturated fatty acid or the C₁₋₆ alkyl ester thereof with a ω-halogenated C₂₋₂₀ fatty acid or a C₁₋₆ alkyl ester thereof to obtain the very-long-chain polyunsaturated fatty acid or the C₁₋₆ alkyl ester thereof,
   wherein in the conversion step, one or more steps selected from (a) to (e) are not comprised: (a) protecting a compound having a hydroxy group into a compound having a protected hydroxy group, (b) deprotecting a compound having a protected hydroxy group into a compound having a hydroxy group, (c) oxidizing an alcohol to an aldehyde or a carboxylic acid, (d) oxidizing an aldehyde to a carboxylic acid, and (e) a step to be performed on a compound derived from the long-chain polyunsaturated fatty acid or the C₁₋₆ alkyl ester thereof in the presence of a stoichiometric amount of a phosphorus compound.
[45] A production method for obtaining a very-long-chain polyunsaturated fatty acid having from 20 to 50 carbon atoms and containing from 3 to 7 double bonds or a C₁₋₆ alkyl ester thereof by converting a long-chain polyunsaturated fatty acid having from 18 to 30 carbon atoms and containing from 3 to 7 double bonds or a C₁₋₆ alkyl ester thereof, the production method comprising, in the conversion step, reacting a Grignard reagent derived from the long-chain polyunsaturated fatty acid or the C₁₋₆ alkyl ester thereof with a ω-halogenated C₂₋₂₀ fatty acid or a C₁₋₆ alkyl ester thereof in the presence of a metal catalyst to obtain the very-long-chain polyunsaturated fatty acid or the C₁₋₆ alkyl ester thereof, wherein in the conversion step, one or more steps selected from (a) to (e) are not comprised: (a) protecting a compound having a hydroxy group into a compound having: a C₁₋₂₀ ether protecting group; a C₃₋₂₀ silyl ether protecting group; or a C₁₋₂₀ ester protecting group; (b) deprotecting a compound having: a C₁₋₂₀ ether protecting group; a C₃₋₂₀ silyl ether protecting group; or a C₁₋₂₀ ester protecting group into a compound having a hydroxy group; (c) oxidizing a compound having a hydroxymethyl group to a compound having a formyl group or a carboxy group; (d) oxidizing a compound having a formyl group to a compound having a carboxy group; and (e) a step to be performed on a compound derived from the long-chain polyunsaturated fatty acid or a C₁₋₆ alkyl ester thereof in the presence of a stoichiometric amount of triphenylphosphine or triphenylphosphonium salt.

### Advantageous Effects of Invention

In one aspect of the present invention, there is provided a method for producing a very-long-chain polyunsaturated fatty acid suitable for efficient production on an industrial scale. In another aspect of the present invention, there is provided a method for producing a synthetic intermediate useful in production of a very-long-chain polyunsaturated fatty acid suitable for efficient production on an industrial scale.

### Description of Embodiments

In the present specification, a very-long-chain polyunsaturated fatty acid is not particularly limited as long as the very-long-chain polyunsaturated fatty acid is a fatty acid having from 20 to 50 carbon atoms and containing a double bond of from 3 to 7. In one aspect of the present invention, the very-long-chain polyunsaturated fatty acid is a fatty acid having 24 or more carbon atoms and containing 3 or more double bonds. In one aspect of the present invention, the very-long-chain polyunsaturated fatty acid has from 24 to 42, from 24 to 40, from 26 to 40, from 28 to 36, from 28 to 40, from 30 to 40, from 24 to 38, from 26 to 38, from 28 to 38, or from 30 to 38 carbon atoms. In one embodiment of the present invention, the very-long-chain polyunsaturated fatty acid has from 3 to 6 or from 4 to 6 double bonds.

In one embodiment of the present invention, the very-long-chain polyunsaturated fatty acid has from 3 to 6 double bonds arranged consecutively with one methylene sandwiched therebetween. In one aspect of the present invention, each double bond is of a cis type (Z configuration). In one embodiment of the present invention, the very-long-chain polyunsaturated fatty acid has from 3 to 6 double bonds arranged consecutively with one methylene sandwiched therebetween in a chemical structure similar to that of docosahexaenoic acid, eicosapentaenoic acid, or arachidonic acid.

In one aspect of the present invention, the very-long-chain polyunsaturated fatty acid is n3 or n6 very-long-chain polyunsaturated fatty acid (n3 VLC-PUFA, n6 VLC-PUFA) containing from 24 to 42 carbons (C24 to C42). Examples thereof include C₂₈₋₃₆: 5n-3 (from 28 to 36 carbons, 5 double bonds, omega-3), C₂₈₋₃₆: 4n-6, C₂₈₋₃₆: 6n-3, and C₂₈₋₃₆: 3n-6. Specific examples thereof include C32: 4n-6, C34: 4n-6, C32: 5n-3, C34: 5n-3, 32: 6n-3, and 34: 6n-3. Preferably, C32: 4n-6 and C34: 5n-3 are exemplified.

In one aspect of the present invention, the very-long-chain polyunsaturated fatty acid, a salt thereof, or an ester thereof is a compound represented by the following formula.

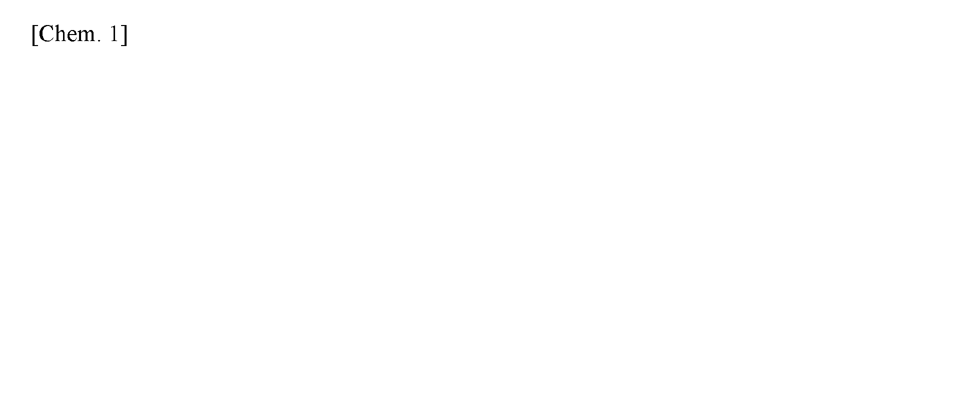

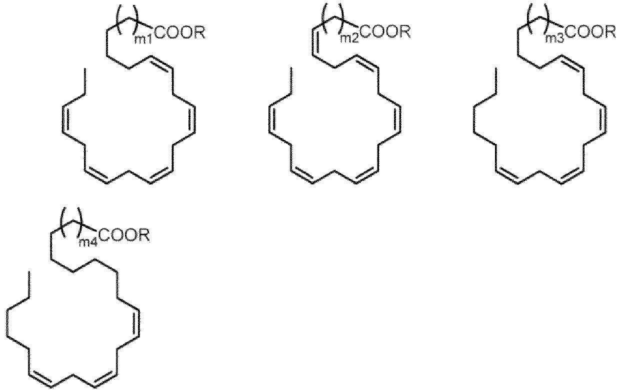

[In the formula, m1, m2, m3 and m4 are independently an integer selected from 4 to 22, from 4 to 20, from 6 to 20, from 8 to 20, from 10 to 20, from 4 to 18, from 6 to 18, from 8 to 18, or from 10 to 18, and R is a hydrogen atom, a cation such as an alkali metal ion, or an ester-forming group such as a C1-6 alkyl.]

In one aspect of the present invention, the very-long-chain polyunsaturated fatty acid can be converted into an ester thereof. The ester conversion can be carried out by a method that is ordinary carried out. Here, the ester is not particularly limited, and includes, for example, a C₁₋₆ alkyl ester, glyceride, and the like. The glyceride may be triglyceride, diglyceride, or monoglyceride, and at least one of constituent fatty acids is the very-long-chain polyunsaturated fatty acid. In one embodiment, the ester of the very-long-chain polyunsaturated fatty acid is an ethyl ester.

In one aspect of the present invention, the very-long-chain polyunsaturated fatty acid can be converted into a salt thereof by treatment with a base. Examples of the salt of the very-long-chain polyunsaturated fatty acid include alkali metal salts such as a potassium salt and a sodium salt.

In one aspect of the present invention, there is provided a method for producing a very-long-chain polyunsaturated fatty acid, an ester thereof, a compound which is a synthetic intermediate useful for the production of the very-long-chain polyunsaturated fatty acid, and is represented by Formula I:

R¹-(CH₂)ₘ-X (I),

[where R¹ is a C₁₈₋₃₀ hydrocarbon group containing from 3 to 7 double bonds,
m is an integer selected from 1 to 19;
X is CO₂H, CO₂R^{a}, or CH₂OR^{b};
R^{a} is a C₁₋₆ alkyl; and
R^{b} is an ether-forming group, a silyl ether-forming group, or an ester-forming group], or a salt of the compound.

The compound represented by Formula I can be produced by a coupling reaction represented by the following method:
a method for producing the compound represented by Formula I, the method comprising reacting a compound represented by Formula II:

   R¹-MgX¹ (II)
[where X¹ is a halogen atom]
with a compound represented by Formula III:

   X²-(CH₂)ₘ-X (III)
[where X² is selected from a halogen atom and a sulfonyloxy group]
to obtain the compound represented by Formula I.

In one aspect of the present invention, there is provided a method for producing the compound of Formula I, which is the very-long-chain polyunsaturated fatty acid or an ester thereof. The compound of Formula I can be produced by a coupling reaction represented by the following method:

A method for producing a compound represented by Formula I:

R¹-(CH₂)ₘ-X (I),

[where R¹ is a C₁₈₋₃₀ hydrocarbon group containing from 3 to 7 double bonds,
m is an integer selected from 1 to 19;
X is CO₂H or CO₂R^{a}; and
R^{a} is a C₁₋₆ alkyl],
the method comprising reacting a compound represented by Formula II:

   R¹-MgX¹ (II)
[where X¹ is a halogen atom]
with a compound represented by Formula III:

   X²-(CH₂)ₘ-X (III)
[where X² is selected from a halogen atom and a sulfonyloxy group]
in a solvent to obtain the compound represented by Formula I.

In one embodiment of the present invention, there is provided the use of the compound of Formula II for the preparation of the very-long-chain polyunsaturated fatty acid or a C₁₋₆ alkyl ester thereof.

In one embodiment of the present invention, there is provided the use of the compound of Formula III for the preparation of the very-long-chain polyunsaturated fatty acid or a C₁₋₆ alkyl ester thereof.

In one aspect of the present invention, a very-long-chain polyunsaturated fatty acid or a C₁₋₆ alkyl ester thereof comprises a compound represented by Formula I:

R¹-(CH₂)ₘ-X (I)

[where R¹ is a C₁₈₋₃₀ hydrocarbon group containing from 3 to 7 double bonds,
m is an integer selected from 1 to 19;
X is CO₂H or CO₂R^{a}; and
R^{a} is a C₁₋₆ alkyl].

In one aspect of the present invention, the compound of Formula II includes a compound represented by Formula II:

R¹-MgX¹ (II)

[where X¹ is a halogen atom].

In one aspect of the present invention, the compound of Formula III includes a compound represented by Formula III:

X²-(CH₂)ₘ-X (III)

[where X² is selected from a halogen atom and a sulfonyloxy group].

In one aspect of the present invention, there is provided a method for producing a compound of Formula Ia, which is a synthetic intermediate useful for producing the very-long-chain polyunsaturated fatty acid. The compound of Formula Ia can be produced by a coupling reaction represented by the following method:

A method for producing a compound represented by Formula Ia:

R¹-(CH₂)ₘ-CH₂OR^{b} (Ia)

[where R¹ is a C₁₈₋₃₀ hydrocarbon group containing from 3 to 7 double bonds;
m is an integer selected from 1 to 19; and
R^{b} is an ether-forming group, a silyl ether-forming group, or an ester-forming group], the method comprising reacting a compound represented by Formula II: R¹-MgX¹ (II)
[where X¹ is a halogen atom]
with a compound represented by Formula IIIa:

   X²-(CH₂)ₘ-CH₂OR^{b} (IIIa)
[where X² is selected from a halogen atom and a sulfonyloxy group]
in a solvent to obtain a compound represented by Formula Ia.

In one embodiment of the present invention, R¹ is a C₁₈, C₂₀ or C₂₂ hydrocarbon group containing from 3 to 6 double bonds. For example, it is a linear hydrocarbon group.

In one aspect of the present invention, R¹ is a group represented by Formula XI:

CH₃-(CH₂-CH=CH)ₐ-(CH₂)_{b}- (XI)

[where a is 5 and b is an integer selected from 2 to 14, or a is 6 and b is an integer selected from 1 to 11], or
a group represented by Formula XII:

   CH₃-(CH₂)₃-(CH₂-CH=CH)_{c}-(CH₂)_{d}- (XII)
[where c is 3 and d is an integer selected from 5 to 17, or c is 4 and d is an integer selected from 2 to 14].

More specifically, R¹ is a substituent represented by Formula XI:

CH₃-(CH₂-CH=CH)ₐ-(CH₂)_{b}- (XI)

[where a is 5 and b is 4, or a is 6 and b is 3], or
a group represented by Formula XII:

   CH₃-(CH₂)₃-(CH₂-CH=CH)_{c}-(CH₂)_{d}- (XII)
[where c is 3 and d is 7, or c is 4 and d is 4].

In one embodiment of the present invention, all double bonds contained in R¹ are of a cis type.

In another embodiment of the present invention, R¹ is a hydrocarbon group selected from 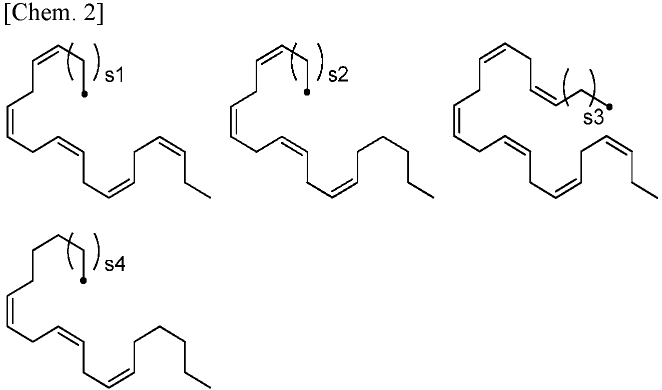 and
[where · represents a bonding point;
s1 is an integer selected from 2 to 14;
s2 is an integer selected from 2 to 14;
s3 is an integer selected from 1 to 11; and
s4 is an integer selected from 2 to 14].

Here, in the present specification, the bonding point indicates a point of bonding to another site in the compound, and the bonding point does not contain methylene (-CH₂-).

More specifically, R¹ is a hydrocarbon group selected from 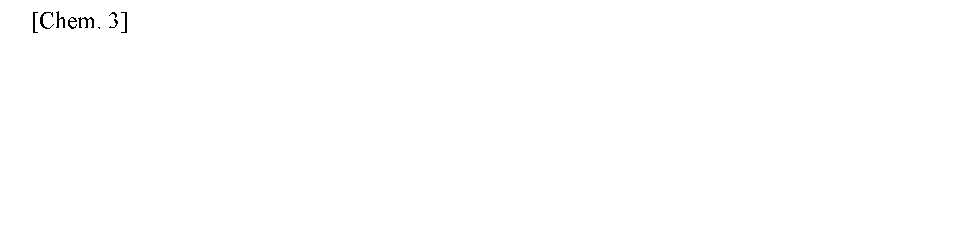 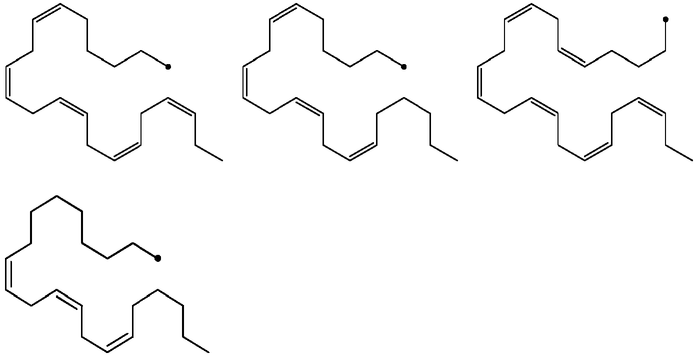 and
[where, · represents a bonding point].

In one embodiment of the present invention, m is from 3 to 17, from 5 to 15, from 7 to 15, or from 7 to 13. In one aspect, m is an odd number. In one aspect, m is 7, 9, 11, or 13.

In the present specification, CH₂OR^{b} represents a structure selected from an ether structure, a silyl ether structure, and an ester structure.

The ether structure is not particularly limited as long as it has a CH₂OC (sp³) structure as CH₂OR^{b}. The ether structure may contain an acetal structure. Examples of the ether structure contain a substituent having from 2 to 20 carbon atoms in the structure (CH₂OR^{b}). Specific examples thereof contain a substituent in which the number of carbon atoms in the structure (CH₂OR^{b}) is from 2 to 20, the number of oxygen atoms is from 1 to 5, and the number of heteroatoms selected from a nitrogen atom, a fluorine atom, a phosphorus atom, a sulfur atom, a chlorine atom, and a bromine atom is from 0 to 3. In one aspect of the present invention, R^{b} is an ether-forming group that forms an ether structure together with adjacent CH₂O, and more specifically, examples of R^{b} include t-butyl, benzyl, p-methoxyphenylbenzyl, trityl, methoxymethyl, methoxyethoxymethyl, and tetrahydropyranyl.

The silyl ether structure is not particularly limited as long as it has a CH₂OSi structure as CH₂OR^{b}. Examples of the silyl ether structure include a substituent having from 4 to 20 carbon atoms in the structure (CH₂OR^{b}). Specific examples thereof include a substituent in which the number of carbon atoms in the structure (CH₂OR^{b}) is from 4 to 20, the number of oxygen atoms is from 1 to 5, the number of silicon atoms is from 1 to 2, and the number of heteroatoms selected from a nitrogen atom, a fluorine atom, a phosphorus atom, a sulfur atom, a chlorine atom, and a bromine atom is from 0 to 3. In one aspect of the present invention, R^{b} is a silyl ether-forming group that forms a silyl ether structure together with adjacent CH₂O, and more specifically, examples of R^{b} include trimethylsilyl, triethylsilyl, t-butyldimethylsilyl, and t-butyldiphenylsilyl.

The ester structure is not particularly limited as long as it has a CH₂OC (=O) structure as CH₂OR^{b}. Examples of the ester structure include a substituent having from 2 to 20 carbon atoms in the structure (CH₂OR^{b}). Specific examples thereof include a substituent in which the number of carbon atoms in the structure (CH₂OR^{b}) is from 2 to 20, the number of oxygen atoms is from 2 to 6, and the number of heteroatoms selected from a nitrogen atom, a fluorine atom, a phosphorus atom, a sulfur atom, a chlorine atom, and a bromine atom is from 0 to 3. In one aspect of the present invention, R^{b} is an ester-forming group that forms an ester structure together with adjacent CH₂O, and more specifically, examples of R^{b} include acetyl, pivaloyl, and benzoyl.

In one embodiment of the present invention, X² of the compound represented by Formula III is selected from a halogen atom and a sulfonyloxy group. In one aspect, examples of the halogen atom include a chlorine atom and a bromine atom. Examples of the sulfonyloxy group in one aspect include a substituent having a -OS (O)₂- structure in which the number of carbon atoms is from 1 to 20, the number of oxygen atoms is from 3 to 5, the number of sulfur atoms is from 1 to 2, and the number of heteroatoms selected from a nitrogen atom, a fluorine atom, a phosphorus atom, a chlorine atom, and a bromine atom is from 0 to 3.

In one embodiment of the present invention, the sulfonyloxy group is a group represented by -OS (O)₂-Q, where Q is a C₁₋₆ alkyl optionally substituted by one or more halogen atoms, for example, one or more fluorine atoms; and a phenyl optionally substituted with from 1 to 3 substituents selected from a C₁₋₃ alkyl or a halogen atom. Specific examples thereof include methanesulfonyloxy, trifluoromethanesulfonyloxy, benzenesulfonyloxy, and toluenesulfonyloxy.

In one aspect of the present invention, X² is a bromine atom.

In one aspect of the present invention, R^{a} is a C₁₋₃ alkyl, for example, methyl or ethyl. In one embodiment, R^{a} is ethyl.

In one aspect of the present invention, X¹ is a halogen atom, for example, a chlorine atom or a bromine atom. In one embodiment, X¹ is a bromine atom.

In one aspect of the present invention, the reaction (coupling reaction) between the compound represented by Formula II and the compound represented by Formula III can be carried out in the presence of a metal catalyst. In one aspect of the present invention, the metal catalyst is not particularly limited as long as it is a metal catalyst capable of obtaining the compound represented by Formula I by the coupling reaction. Examples thereof include a copper catalyst, a nickel catalyst, a palladium catalyst, and an iron catalyst. In one embodiment of the present invention, the metal catalyst is a copper catalyst.

The copper catalyst is not particularly limited as long as it is a catalyst containing a copper atom in the catalyst. Examples thereof include lithium tetrachlorocuprate (Li₂CuCl₄), copper (I) iodide (CuI), copper (I) bromide (CuBr), copper (I) chloride (CuCl), copper (II) bromide (CuBr₂), copper (II) chloride (CuCl₂), copper (I) cyanide (CuCN), a combination of copper (II) chloride and lithium chloride in a molar ratio of 1:2 (CuCl₂ · 2LiCl), a combination of copper (I) cyanide and lithium chloride in a molar ratio of 1:2 (CuCN · 2LiCl), a copper (I) bromide-dimethyl sulfide complex (CuBr/CH₃SCH₃), copper (II) acetylacetonate (Cu (acac)₂), and copper (II) acetate (Cu (OAc)₂). The combination of lithium chloride and copper (II) chloride and the combination of lithium chloride and copper (I) cyanide may be added into the reaction system after the two are mixed, or may be added separately into the reaction system. In one embodiment of the present invention, the copper catalyst is lithium tetrachlorocuprate. The lithium tetrachlorocuprate is a double salt of lithium chloride-copper (II) chloride (Li₂CuCl₄). In one embodiment, the lithium tetrachlorocuprate that can be purchased as a THF solution or the like can be used.

The nickel catalyst is not particularly limited as long as it is a catalyst containing a nickel atom in the catalyst. Examples thereof include nickel (II) chloride (NiCl₂) and nickel (II) acetylacetonate (Ni(acac)₂).

The palladium catalyst is not particularly limited as long as it is a catalyst containing a palladium atom in the catalyst. Examples thereof include palladium (II) acetate (Pd (OAc)₂), tetrakistriphenylphosphine palladium (0) (Pd(PPh₃)₄), palladium (II) acetylacetonate (Pd(acac)₂), and palladium (II) chloride (PdCl₂).

The iron catalyst is not particularly limited as long as it is a catalyst containing an iron atom in the catalyst. Examples thereof include iron (III) acetylacetonate (Fe(acac)₃) and iron (III) chloride (FeCl₃).

The amount of the metal catalyst is not particularly limited as long as it is a catalytic amount with respect to the compound represented by Formula III, and is, for example, from 0.1 to 20 mol%, from 0.1 to 10 mol%, from 0.5 to 20 mol%, from 0.5 to 10 mol%, from 0.5 to 7 mol%, from 1 to 10 mol%, from 1 to 7 mol%, or from 3 to 6 mol%.

In one aspect of the present invention, the reaction (coupling reaction) between the compound represented by Formula II and the compound represented by Formula III can be carried out in the presence of an additive. In one embodiment of the present invention, the additive is not particularly limited as long as the additive does not adversely affect the coupling reaction, improves a yield of the compound represented by Formula I, and/or shortens a reaction time as compared with a case where the additive is not added. Examples thereof include a nitrogen-based additive, an unsaturated hydrocarbon-based additive, a silyl-based additive, and a combination thereof. In one aspect, the additive is a nitrogen-based additive.

The nitrogen-based additive is not particularly limited as long as it is a compound having a nitrogen atom in the compound. Specific examples thereof include a compound in which the number of carbon atoms is from 4 to 20, the number of nitrogen atoms is from 1 to 5, and the number of heteroatoms selected from an oxygen atom, a fluorine atom, a silicon atom, a phosphorus atom, a sulfur atom, a chlorine atom, and a bromine atom is from 0 to 5. More specifically, examples of the nitrogen-based additive include lactam-based additives such as N-methylpyrrolidone (NMP); urea-based additives such as N,N'-dimethylpropyleneurea (DMPU); phosphoric acid amide-based additives such as hexamethylphosphoric acid triamide (HMPA); and amine-based additives such as hexamethylenetetramine (HMTA) and tetramethylethylenediamine (TMEDA). In one embodiment of the present invention, the nitrogen-based additive is a lactam-based additive, for example, N-methylpyrrolidone (NMP).

The unsaturated hydrocarbon-based additive is not particularly limited as long as it is a hydrocarbon compound having a carbon-carbon unsaturated bond (double bond, triple bond) in the compound. Specific examples thereof include a compound in which the number of carbon atoms is from 4 to 20, the number of carbon-carbon unsaturated bonds is from 1 to 6, and the number of hetero atoms selected from a nitrogen atom, an oxygen atom, a fluorine atom, a silicon atom, a phosphorus atom, a sulfur atom, a chlorine atom, and a bromine atom is from 0 to 5. More specifically, examples of the unsaturated bond-based additive include 1,3-diene-based additives such as 1,3-butadiene and isoprene; and aromatic additives such as 1-phenylpropyne.

The silyl-based additive is not particularly limited as long as it is a compound having a silicon atom in the compound. Specific examples thereof include a compound in which the number of carbon atoms is from 2 to 20, the number of silicon atoms is from 1 to 2, and the number of hetero atoms selected from a nitrogen atom, an oxygen atom, a fluorine atom, a phosphorus atom, a sulfur atom, a chlorine atom, and a bromine atom is from 0 to 5. More specifically, examples of the silyl-based additive include chlorotrimethylsilane (TMS-Cl).

In one embodiment of the present invention, examples of the additive include N-methylpyrrolidone (NMP), N, N'-dimethylpropyleneurea (DMPU), hexamethylphosphoric acid triamide (HMPA), hexamethylenetetramine (HMTA), tetramethylethylenediamine (TMEDA), 1,3-butadiene, isoprene, 1-phenylpropyne, and chlorotrimethylsilane (TMS-Cl). In one embodiment of the present invention, the additive is N-methylpyrrolidone (NMP). N-methylpyrrolidone is available by purchase, and a reagent of high purity purchased may be used as it is, or may be purified by distillation before use.

In one embodiment of the present invention, an amount of the additive is from 0.1 to 10 equivalents, from 0.1 to 5 equivalents, from 1 to 10 equivalents, or from 1 to 5 equivalents with respect to the compound represented by Formula III.

In one aspect of the present invention, the reaction (coupling reaction) between the compound represented by Formula II and the compound represented by Formula III can be performed in a solvent. The usable solvent may be any solvent as long as it does not adversely affect the reaction, and examples thereof include aliphatic hydrocarbons such as hexane and heptane; aromatic hydrocarbons such as benzene, toluene, and xylene; ethers such as diethyl ether, diisopropyl ether, tetrahydrofuran, dioxane, dimethoxyethane, diethylene glycol dimethyl ether, and cyclopentyl methyl ether; and nitriles such as acetonitrile. In one embodiment of the present invention, the reaction is carried out in a solvent selected from ethers such as diethyl ether, diisopropyl ether, tetrahydrofuran, dioxane, dimethoxyethane, diethylene glycol dimethyl ether, and cyclopentyl methyl ether, or a mixed solvent of two or more solvents.

In one embodiment of the present invention, the order of mixing of compounds in the coupling reaction is not particularly limited. In one embodiment of the present invention, in a solvent (optionally in the presence of a metal catalyst and/or an additive),
a mixture containing the compound represented by Formula II:

   R¹-MgX¹ (II)
can be added to the compound represented by Formula III:

   X²-(CH₂)ₘ-X (III).

Examples of mixtures containing the compound of Formula II include a mixture containing the compound of Formula II and a solvent. Examples of the solvent include solvents usable in the coupling reaction. In one embodiment, the solvent is the same as the solvent of the coupling reaction.

In one embodiment of the present invention, when X in the compound represented by Formula III is CO₂R^{a} or CH₂OR^{b}, the amount of the compound represented by Formula II used in the reaction is from 1 to 5 equivalents, from 1 to 3 equivalents, from 1.5 to 5 equivalents, or from 1.5 to 3 equivalents with respect to the compound represented by Formula III.

In one embodiment of the present invention, when X in the compound represented by Formula III is CO₂H, the amount of the compound represented by Formula II used in the reaction is from 1 to 5 equivalents, from 2 to 5 equivalents, from 1 to 3 equivalents, from 2 to 3 equivalents, or from 2.5 to 3 equivalents with respect to the compound represented by Formula III.

In one embodiment of the present invention, a time for adding the mixture containing the compound of Formula II is from 5 minutes to 80 minutes, from 5 minutes to 30 minutes, or from 5 minutes to 10 minutes.

In one embodiment, a rate of adding the mixture containing the compound of Formula II is from 0.1 mL/min to 1000 mL/min, from 0.1 mL/min to 200 mL/min, from 0.1 mL/min to 100 mL/min, or from 0.3 mL/min to 20 mL/min.

In one embodiment of the present invention, the reaction of the compound of Formula II and the compound of Formula III can be performed at a reaction temperature of from -20°C to 35°C, from -20°C to 25°C, from -10°C to 35°C, from -10°C to 25°C, from -10°C to 10°C, from 0°C to 25°C, or from 0°C to 10°C.

In one embodiment of the present invention, the reaction of the compound of Formula II and the compound of Formula III can be performed under an inert gas atmosphere, for example, a nitrogen atmosphere or an argon atmosphere.

In one aspect of the present invention, the compound represented by Formula II:

R¹-MgX¹ (II)

can be obtained by, for example, converting, in a solvent, the compound represented by Formula IIc:

R¹-X¹ (IIc).

Specifically, the compound represented by Formula II can be obtained by, for example, reacting, in a solvent, the compound represented by Formula IIc:

R¹-X¹ (IIc)

with magnesium. In one embodiment of the present invention, the reaction can be carried out in the presence of an activator selected from iodine and 1,2-dibromoethane. Specifically, the compound represented by Formula IIc or a mixture containing the compound can be added in the presence of magnesium (optionally an activator selected from iodine and 1,2-dibromoethane) in a solvent to convert the compound represented by Formula IIc into the compound represented by Formula II. Here, examples of mixtures containing the compound of Formula IIc include a mixture containing the compound of Formula IIc and a solvent. Examples of the solvent include solvents that can be used in a reaction to convert the compound of Formula IIc into the compound of Formula II. In one embodiment, the solvent is the same as the solvent of the reaction.

In one embodiment, the compound represented by Formula II can be obtained by reacting the compound represented by Formula IIc:

R¹-X¹ (IIc)

with the compound represented by Formula IId:

   R³-MgX⁴ (IId)
[where R³ is selected from an isopropyl group and a secondary butyl group, and X⁴ is selected from a bromine atom and a chlorine atom]
or a lithium chloride complex thereof. Specifically, the compound represented by Formula IIc and the compound represented by Formula IId can be mixed in a solvent to be converted into the compound represented by Formula II.

In the reaction for converting the compound of Formula IIc into the compound of Formula II, the usable solvent may be any solvent as long as it does not adversely affect the reaction, and examples thereof include aliphatic hydrocarbons such as hexane and heptane; aromatic hydrocarbons such as benzene, toluene, and xylene; and ethers such as diethyl ether, diisopropyl ether, tetrahydrofuran, dioxane, dimethoxyethane, diethylene glycol dimethyl ether, and cyclopentyl methyl ether. In one embodiment of the present invention, the reaction is carried out in a solvent selected from ethers such as diethyl ether, diisopropyl ether, tetrahydrofuran, dioxane, dimethoxyethane, diethylene glycol dimethyl ether, and cyclopentyl methyl ether, or a mixed solvent of two or more solvents.

In one embodiment of the present invention, an amount of magnesium is from 1 to 10 equivalents, from 2 to 10 equivalents, from 1 to 6 equivalents, or from 2 to 6 equivalents with respect to the compound represented by Formula IIc. Amounts of iodine and 1,2 dibromoethane are from 0.01 to 0.1 equivalents, from 0.01 to 0.05 equivalents, from 0.03 to 0.1 equivalents, or from 0.03 to 0.05 equivalents with respect to magnesium.

The amount of the compound represented by Formula IId or the lithium chloride complex thereof is from 1 to 3 equivalents, from 1 to 2 equivalents, from 1.1 to 3 equivalents, or from 1.1 to 2 equivalents with respect to the compound represented by Formula IIc. An amount of lithium chloride is from 0.5 to 2 equivalents, from 0.5 to 1.5 equivalents, from 1 to 1.5 equivalents, or from 1 to 1.2 equivalents with respect to magnesium.

In one embodiment of the present invention, the time for adding the compound of Formula IIc or the mixture containing the compound is from 5 minutes to 60 minutes, from 5 minutes to 30 minutes, or from 10 minutes to 30 minutes.

In one embodiment, the rate of adding the compound of Formula IIc or the mixture containing the compound is from 0.1 mL/min to 1000 mL/min, from 0.1 mL/min to 200 mL/min, from 0.1 mL/min to 100 mL/min, or from 0.5 mL/min to 20 mL/min.

In one aspect of the present invention, the present conversion can be performed at a reaction temperature of from -40°C to 80°C, from -40°C to 60°C, from -20°C to 80°C, from -20°C to 60°C, from 0°C to 60°C, or from 20°C to 60°C. In one aspect, this conversion can be performed under an inert gas atmosphere, for example, a nitrogen atmosphere or an argon atmosphere.

The compound represented by Formula II may be produced in the reaction system of the coupling reaction, or may be separately produced and added to the reaction system.

In one embodiment of the present invention, the compound represented by Formula IIc:

R⁴-CH₂-X¹ (IIc)

[where R⁴ is a C₁₇₋₂₉ hydrocarbon group containing from 3 to 7 double bonds and X¹ is a halogen atom]
can be produced, for example, from a compound of Formula IV by the following method.

(1) A compound represented by Formula IV:

   R⁴-CO₂R² (IV)

   [where R² is a hydrogen atom or an ester-forming group such as a C₁₋₆ alkyl]
   is converted into a compound represented by Formula IIa:

      R⁴-CH₂-OH (IIa);
(2) the compound represented by Formula IIa is converted into a compound represented by Formula IIb:

   R⁴-CH₂-OX³ (IIb)

   [where X³ is a sulfonyl group]; and
(3) the compound represented by Formula IIb is converted into the compound represented by Formula IIc

   R⁴-CH₂-X¹ (IIc)

   [where X¹ is a halogen atom].

In one embodiment of the present invention, the compound represented by Formula IIc:

R¹-X¹ (IIc)

can be produced, for example, from the compound of Formula IIa by the following method.

(2) The compound represented by Formula IIa:

R¹-OH (IIa)

is converted into the compound represented by Formula 11b:

   R¹-OX³ (IIb)
[where X³ is a sulfonyl group]; and

(3) the compound represented by Formula IIb is converted into the compound represented by Formula IIc:

R¹-X¹ (IIc).

(1) In one embodiment of the present invention, conversion of the compound of Formula IV into the compound of Formula IIa can be performed by reacting the compound of Formula IV with a reducing agent in a solvent.

As the reducing agent, a reducing agent for converting carboxylic acid and/or ester ordinary used by those skilled in the art into alcohol can be used, and examples thereof include Red-Al (trade name) (sodium bis(2-methoxyethoxy) aluminum hydride), lithium aluminum hydride, diisobutylaluminum hydride, and lithium borohydride. More preferably, Red-Al (trade name) having no spontaneous ignition property is exemplified. The usable solvent may be any solvent as long as it does not adversely affect the reaction, and examples thereof include aliphatic hydrocarbons such as hexane and heptane; aromatic hydrocarbons such as benzene, toluene, and xylene; and ethers such as diethyl ether, diisopropyl ether, tetrahydrofuran, dioxane, dimethoxyethane, diethylene glycol dimethyl ether, and cyclopentyl methyl ether. In one embodiment of the present invention, the reaction is carried out in a solvent selected from ethers such as diethyl ether, diisopropyl ether, tetrahydrofuran, dioxane, dimethoxyethane, diethylene glycol dimethyl ether, and cyclopentyl methyl ether, or a mixed solvent of two or more solvents.

In one embodiment of the present invention, an amount of the reducing agent is from 0.1 to 4 equivalents, from 0.5 to 4 equivalents, from 0.1 to 2 equivalents, or from 0.5 to 2 equivalents with respect to the compound represented by Formula IV

In one aspect, the present conversion can be performed at a reaction temperature of from - 20°C to 60°C, from -20°C to 40°C, from 0°C to 60°C, from 0°C to 40°C, or from 0°C to 25°C. In one aspect, this conversion can be performed under an inert gas atmosphere, for example, a nitrogen atmosphere or an argon atmosphere.

(2) In one embodiment of the present invention, conversion of the compound of Formula IIa into the compound of Formula IIb can be performed by reacting the compound of Formula IIa with a sulfonylating agent in a solvent in the presence of a base.

The sulfonyl group represented by X³ is not particularly limited as long as it is a substituent that forms an oxysulfonyl structure (-OSO₂- structure) together with an adjacent oxygen atom. Examples of the sulfonyl group include a substituent having a -SO₂- structure in which the number of carbon atoms is from 1 to 20, the number of oxygen atoms is from 2 to 4, the number of sulfur atoms is from 1 to 2, and the number of heteroatoms selected from a nitrogen atom, a fluorine atom, a phosphorus atom, a chlorine atom, and a bromine atom is from 0 to 3. Specific examples thereof include methanesulfonyl, trifluoromethanesulfonyl, benzenesulfonyl, and toluenesulfonyl. In one aspect of the present invention, the sulfonyl group is toluenesulfonyl (p-toluenesulfonyl or the like).

In one embodiment of the present invention, the sulfonyl group is a group represented by -S (O)₂-Q, where Q is a C₁₋₆ alkyl optionally substituted by one or more halogen atoms, for example, a fluorine atom; and a phenyl optionally substituted with from 1 to 3 substituents selected from a C₁₋₃ alkyl or a halogen atom. Specific examples thereof include methanesulfonyl, trifluoromethanesulfonyl, benzenesulfonyl, and toluenesulfonyl.

As the sulfonylating agent, a sulfonylating agent corresponding to the sulfonyl group represented by X³ ordinary used by those skilled in the art can be used. Examples thereof include methanesulfonyl chloride (MsCl), trifluoromethanesulfonic anhydride (Tf₂O), benzenesulfonyl chloride (BsCl), toluenesulfonyl chloride (TsCl) (p-toluenesulfonyl chloride (p-TsCl), etc.), methanesulfonic anhydride (Ms₂O), and toluenesulfonic anhydride (Ts₂O). More preferred examples thereof include trifluoromethanesulfonic anhydride, toluenesulfonyl chloride (p-toluenesulfonyl chloride or the like), methanesulfonic anhydride, and toluenesulfonic anhydride which are not specified as toxic agents. More preferred examples thereof include toluenesulfonyl chloride (TsCl) (p-toluenesulfonyl chloride (p-TsCl) and the like). As the base, a known compound can be used, and examples thereof include amines. Examples of the amines include tertiary amines, heterocyclic aromatic amines, and combinations thereof. Examples of the tertiary amine include trialkylamines (for example, tri(C₁₋₆ alkyl) amine) such as triethylamine, trimethylamine, and diisopropylethylamine, and dialkylmonoarylamines (for example, di(C₁₋₆ alkyl) mono C₆₋₁₀ arylamine) such as dimethylaniline and diethylaniline. Examples of the heterocyclic aromatic amine include heterocyclic aromatic amines (for example, a monocyclic heterocyclic aromatic amine having from 1 to 2 nitrogen atoms as ring-constituting atoms) having a nitrogen atom as a ring-constituting atom such as pyridine, 4-dimethylaminopyridine, and imidazole. In one embodiment, the base is a trialkylamine, specifically a combination of triethylamine and trimethylamine. The usable solvent may be any solvent as long as it does not adversely affect the reaction, and examples thereof include aliphatic hydrocarbons such as hexane and heptane; aromatic hydrocarbons such as benzene, toluene, and xylene; ethers such as diethyl ether, diisopropyl ether, tetrahydrofuran, dioxane, dimethoxyethane, diethylene glycol dimethyl ether, and cyclopentyl methyl ether; nitriles such as acetonitrile; and alkyl halides such as dichloromethane. In one embodiment of the present invention, the reaction is carried out in a solvent selected from ethers such as diethyl ether, diisopropyl ether, tetrahydrofuran, dioxane, dimethoxyethane, diethylene glycol dimethyl ether, and cyclopentyl methyl ether, or a mixed solvent of two or more solvents.

In one embodiment of the present invention, an amount of the sulfonylating agent is from 1 to 4 equivalents, from 1 to 2.5 equivalents, from 1.2 to 4 equivalents, or from 1.2 to 2.5 equivalents with respect to the compound represented by Formula IIa. In one embodiment of the present invention, an amount of the base is from 0.1 to 5 equivalents, from 0.1 to 3 equivalents, from 1 to 5 equivalents, or from 1 to 3 equivalents with respect to the compound represented by Formula IIa.

In one aspect of the present invention, the present conversion can be performed at a reaction temperature of from 0°C to 60°C, from 0°C to 40°C, from 20°C to 60°C, from 20°C to 40°C, or from 20°C to 30°C. In one aspect, this conversion can be performed under an inert gas atmosphere, for example, a nitrogen atmosphere or an argon atmosphere.

(3) In one aspect of the present invention, conversion of the compound of Formula IIb into the compound of Formula IIc can be performed by reacting the compound of Formula IIb with a halogenating agent in a solvent.

As the halogenating agent, a halide salt corresponding to the halogen atom represented by X¹ ordinary used by those skilled in the art can be used. For example, when X¹ is a chlorine atom, examples of the halogenating agent (chlorinating agent) include lithium chloride (LiCl), sodium chloride (NaCl), and tetrabutylammonium chloride (TBACl). In one embodiment, the halogenating agent (chlorinating agent) is lithium chloride (LiCl). When X¹ is a bromine atom, examples of the halogenating agent (brominating agent) include lithium bromide (LiBr), sodium bromide (NaBr), and tetrabutylammonium bromide (TBABr). In one embodiment, the halogenating agent (brominating agent) is lithium bromide (LiBr). When X¹ is an iodine atom, examples of the halogenating agent (iodinating agent) include sodium iodide (NaI). The usable solvent may be any solvent as long as it does not adversely affect the reaction, and examples thereof include aliphatic hydrocarbons such as hexane and heptane; aromatic hydrocarbons such as benzene, toluene, and xylene; ethers such as diethyl ether, diisopropyl ether, tetrahydrofuran, dioxane, dimethoxyethane, diethylene glycol dimethyl ether, and cyclopentyl methyl ether; nitriles such as acetonitrile; and ketones such as acetone. In one embodiment of the present invention, the reaction is carried out in a solvent selected from ethers such as diethyl ether, diisopropyl ether, tetrahydrofuran, dioxane, dimethoxyethane, diethylene glycol dimethyl ether, and cyclopentyl methyl ether, or ketones such as acetone, or a mixed solvent of two or more solvents.

In one embodiment of the present invention, an amount of the halogenating agent is from 1 to 10 equivalents, from 1 to 5 equivalents, from 2 to 10 equivalents, or from 2 to 5 equivalents with respect to the compound represented by Formula IIb.

In one aspect of the present invention, the present conversion can be performed at a reaction temperature of from 20°C to 80°C, from 20°C to 60°C, from 40°C to 80°C, or from 40°C to 60°C. In one aspect, this conversion can be performed under an inert gas atmosphere, for example, a nitrogen atmosphere or an argon atmosphere.

In one aspect of the present invention, two or more successive conversions can be performed as a one-pot synthesis using the same reaction vessel. In one embodiment of the present invention, (2) conversion of the compound of Formula IIa into the compound of Formula IIb and (3) conversion of the compound of Formula IIb into the compound of Formula IIc can be carried out as the one-pot synthesis.

In the present specification, the one-pot synthesis may be a method in which the subsequent conversion is consecutively performed without isolating and purifying an intermediate product produced in the previous conversion using the same reaction vessel. The number of conversions consecutively performed is not particularly limited as long as it is two or more.

In one embodiment of the present invention, specific examples of the one-pot synthesis for preparing the compound represented by Formula IIc include a one-pot synthesis comprising (2) reacting the compound represented by Formula IIa with TsCl in the presence of one or two or more trialkylamines in a solvent to be converted into the compound represented by Formula IIb:

R⁴-CH₂-OX³ (IIb)

[where X³ is toluenesulfonyl]; and (3) reacting the compound represented by Formula IIb with LiBr without isolation, and preparing the compound represented by Formula IIc:

   R⁴-CH₂-X¹ (IIc)
[where X¹ is a bromine atom].

In one embodiment of the present invention, as the compound of Formula IV, C₁₈₋₂₂ polyunsaturated fatty acids, such as C22: 6n-3 (DHA), C20: 5n-3 (EPA), C20: 4n-6 (arachidonic acid), C20: 3n-6 (dihomo-γ-linolenic acid (DGLA)), or esters thereof, specifically C₁₋₆ alkyl esters thereof, such as ethyl esters, can be used.

In one aspect of the present invention, the compound of Formula I where X is CH₂OR^{b} can be converted into an alcohol in a manner ordinary used by those skilled in the art. When CH₂OR^{b} has the ether structure that forms an acetal structure, for example, the compound can be converted into an alcohol by using a Brønsted acid such as trifluoroacetic acid or hydrochloric acid. When CH₂OR^{b} has the silyl ether structure, the compound can be converted into an alcohol compound by using a fluoride ion source such as potassium fluoride or tetrabutylammonium fluoride. The resulting alcohol is a compound represented by Formula V:

R¹-(CH₂)ₘ-CH₂OH (V).

In one aspect of the present invention, the oxidation of the compound of Formula V can be carried out by an oxidation reaction ordinary performed by those skilled in the art to convert an alcohol into a carboxylic acid. As the oxidizing agent, for example, a Jones reagent and reagents such as a combination of 2,2,6,6-tetramethylpiperidine-1-oxyl (TEMPO), sodium hypochlorite and sodium chlorite can be used. The resulting very-long-chain polyunsaturated fatty acid is a compound represented by Formula Ib:

R¹-(CH₂)ₘ-CO₂H (Ib).

In one aspect of the present invention, a fatty acid of Formula Ib can be converted to obtain a C₁₋₆ alkyl ester, for example, an ethyl ester. The esterification reaction can be carried out under conditions ordinary performed by those skilled in the art.

In one aspect of the present invention, synthetic intermediates such as the compound represented by Formula IIc:

R⁴-CH₂-X¹ (IIc)

{e.g., (3E, 6E, 9E, 12E, 15E)-20-bromo-3,6,9,12,15-eicosapentaene (CAS Registry Number: 181213-47-8), (3E, 6E, 9E, 12E, 15E)-20-chloro-3,6,9, 12,15-eicosapentaene (CAS Registry

Number: 2519469-69-1)} and the compound represented by Formula III:

X²-(CH₂)ₘ-X (III)

can be obtained by preparation by a known method or purchase.

In one embodiment of the present invention, there is provided a production method for obtaining a very-long-chain polyunsaturated fatty acid (VLC-PUFA) having from 20 to 50 carbon atoms and containing from 3 to 7 double bonds or a C₁₋₆ alkyl ester thereof by converting a long-chain polyunsaturated fatty acid (LC-PUFA) having from 18 to 30 carbon atoms and containing from 3 to 7 double bonds or a C₁₋₆ alkyl ester thereof, the production method comprising, in the conversion step, reacting a Grignard reagent derived from a long-chain polyunsaturated fatty acid or a C₁₋₆ alkyl ester thereof with a ω-halogenated C₂₋₂₀ fatty acid or a C₁₋₆ alkyl ester thereof to obtain a very-long-chain polyunsaturated fatty acid or a C₁₋₆ alkyl ester thereof, and the method not comprising, in the conversion step: (a) protecting a compound having a hydroxy group into a compound having a protected hydroxy group, (b) deprotecting a compound having a protected hydroxy group into a compound having a hydroxy group, (c) oxidizing an alcohol to an aldehyde or a carboxylic acid, (d) oxidizing an aldehyde to a carboxylic acid, or (e) a step to be performed on a compound derived from a long-chain polyunsaturated fatty acid or a C₁₋₆ alkyl ester thereof in the presence of a stoichiometric amount of a phosphorus compound.

**In** one aspect of the present invention, there is provided the method that does not comprise, in the conversion step, (a) protecting a compound having a hydroxy group into a compound having a C₁₋₂₀ ether protecting group, a C₃₋₂₀ silyl ether protecting group, or a C₁₋₂₀ ester protecting group; (b) deprotecting a compound having a C₁₋₂₀ ether protecting group, a C₃₋₂₀ silyl ether protecting group or a C₁₋₂₀ ester protecting group into a compound having a hydroxy group; (c) oxidizing a compound having a hydroxymethyl group to a compound having a formyl group or a carboxy group; (d) oxidizing a compound having a formyl group to a compound having a carboxy group; or (e) a step to be performed on a compound derived from the long-chain polyunsaturated fatty acid or a C₁₋₆ alkyl ester thereof in the presence of a stoichiometric amount of triphenylphosphine or triphenylphosphonium salt.

Here, the production method that does not comprise steps (a), (b), (c), (d), and/or step (e) in the conversion step means that the step (a) or the like is not comprised in all of one or more conversion steps of converting LC-PUFA or an alkyl ester thereof to produce VLC-PUFA or an alkyl ester thereof. Meanwhile, steps other than the conversion step may comprise the step (a) and the like.

For example, it means that when the compound represented by Formula Ia:

R¹-(CH₂)ₘ-CH₂OR^{b} (Ia)

is produced to be deprotected and oxidized, and thus to produce a VLC-PUFA, (b) the step of deprotection to a hydroxy group and (c) the step of oxidation of an alcohol to a carboxylic acid are comprised in the conversion step.

Meanwhile, for example, it does not mean that when the compound represented by Formula IIIa:

X²-(CH₂)ₘ-CH₂OR^{b} (IIIa)

is deprotected and oxidized to be converted into a compound represented by Formula IIIb:

   X²-(CH₂)ₘ-CO₂H (IIIb),

   and
the compound of Formula IIIb is used in a coupling reaction with the compound of Formula II, (b) the deprotection to a hydroxy group and (c) the oxidation step of an alcohol to a carboxylic acid are comprised in the conversion step. That is, the step of converting the compound of Formula IIIa into the compound of Formula IIIb is not comprised in one or more conversion steps that convert LC-PUFA or an alkyl ester thereof to produce VLC-PUFA or an alkyl ester thereof.

In one aspect of the present invention, as a long-chain polyunsaturated fatty acid having from 18 to 30 carbon atoms and containing from 3 to 7 double bonds or a C₁₋₆ alkyl ester thereof, the compound represented by Formula IV:

R⁴-CO₂R² (IV)

[where R⁴ is a C₁₇₋₂₉ hydrocarbon group containing from 3 to 7 double bonds and R² is a hydrogen atom or C₁₋₆ alkyl]
or a long-chain polyunsaturated fatty acid or an alkyl ester thereof as described herein is exemplified.

In one embodiment of the present invention, as the very-long-chain polyunsaturated fatty acid having from 20 to 50 carbon atoms and containing from 3 to 7 double bonds or a C₁₋₆ alkyl ester thereof, the compound represented by Formula I:

R¹-(CH₂)ₘ-X (I)

[where R¹ is a C₁₈₋₃₀ hydrocarbon group containing from 3 to 7 double bonds,
m is an integer selected from 1 to 19;
X is CO₂H or CO₂R^{a}; and
R^{a} is a C₁₋₆ alkyl]
or the very-long-chain polyunsaturated fatty acid or an alkyl ester thereof as described herein is exemplified.

In one aspect of the present invention, in a reaction between a Grignard reagent derived from a long-chain polyunsaturated fatty acid or a C₁₋₆ alkyl ester thereof and a ω-halogenated C₂₋₂₀ fatty acid or a C₁₋₆ alkyl ester thereof, as the Grignard reagent derived from the long-chain polyunsaturated fatty acid or the C₁₋₆ alkyl ester thereof,
the compound represented by Formula II:

   R¹-MgX¹ (II)
[where R¹ is a C₁₈₋₃₀ hydrocarbon group containing from 3 to 7 double bonds, and X¹ is a halogen atom]
or a Grignard reagent as described herein is exemplified.

In one embodiment of the present invention, as the ω-halogenated C₂₋₂₀ fatty acid or the C₁₋₆ alkyl ester thereof, the compound represented by Formula III:

X²-(CH₂)ₘ-X (III)

[where X² is a halogen atom;
m is an integer selected from 1 to 19;
X is CO₂H or CO₂R^{a}; and
R^{a} is a C₁₋₆ alkyl]
or the ω-halogenated fatty acid or an alkyl ester thereof as described herein is exemplified.

In one embodiment of the present invention, the reaction can be carried out in the presence of a metal catalyst. Examples of the metal catalyst include the metal catalyst those described herein.

In one aspect of the present invention, the reaction can be carried out in the presence of an additive. Examples of the additive include the additive those described herein.

In one aspect of the present invention, examples of the protected hydroxy group include a hydroxy group protected with a protective group ordinary used by those skilled in the art to protect a hydroxy group. In one embodiment, the protected hydroxy group is a group represented by -O-R^{C}, and examples thereof include a protected hydroxy group in which -OR^{C} forms an ether structure having a -O-C(sp3) structure, a protected hydroxy group in which -O-R^{C} forms a silyl ether structure having a -O-Si structure, and a protected hydroxy group in which -O-R^{C} forms an ester structure having a -O-C (=O) structure.

In one aspect of the present invention, examples of the protected hydroxy group forming an ether structure, the protected hydroxy group forming a silyl ether structure, the protected hydroxy group forming an ester structure, the ether protecting group, the silyl ether protecting group, and the ester protecting group in the protection step of (a) and the deprotection step of (b) comprise a protected hydroxy group forming a C₁₋₂₀ ether structure, a protected hydroxy group forming a C₃₋₂₀ silyl ether structure, a protected hydroxy group forming a C₁₋₂₀ ester structure, a C₁₋₂₀ ether protecting group, a C₃₋₂₀ silyl ether protecting group, and a C₁₋₂₀ ester protecting group, respectively. Here, the number of carbon atoms of these substituents is the number of carbon atoms of a portion substituted for a hydrogen atom of a hydroxy group, and for example, the number of carbon atoms of t-butyl ether is 4.

Examples of the C₁₋₂₀ ether protecting group include t-butyl, benzyl, p-methoxyphenylbenzyl, trityl, methoxymethyl, methoxyethoxymethyl, and tetrahydropyranyl. The protected hydroxy groups formed by these protecting groups are t-butyloxy, benzyloxy, p-methoxyphenylbenzyloxy, trityloxy, methoxymethyloxy, methoxyethoxymethyloxy, and tetrahydropyranyloxy. These groups form t-butyl ether, benzyl ether, p-methoxyphenyl benzyl ether, trityl ether, methoxymethyl ether, methoxyethoxymethyl ether, tetrahydropyranyl ether.

Examples of the C₃₋₂₀ silyl ether protecting group include trimethylsilyl, triethylsilyl, t-butyldimethylsilyl, and t-butyldiphenylsilyl. The protected hydroxy groups formed by these protecting groups are trimethylsilyloxy, triethylsilyloxy, t-butyldimethylsilyloxy, and t-butyldiphenylsilyloxy. These groups form trimethylsilyl ether, triethylsilyl ether, t-butyldimethylsilyl ether, and t-butyldiphenylsilyl ether.

Examples of the C₁₋₂₀ ester protecting group include acetyl, pivaloyl, and benzoyl. The protected hydroxy group formed by these protecting groups is acetoxy, pivaloyloxy, or benzoyloxy. These groups form acetate, pivalate, and benzoate ester.

In one aspect of the present invention, the oxidation conditions in (c) the step of oxidizing an alcohol (hydroxymethyl group) or (d) the step of oxidizing an aldehyde (formyl group) are not particularly limited as long as the oxidation conditions are conditions for conversion into an aldehyde (formyl group), a carboxylic acid (carboxy group), or a carboxylic acid (carboxy group), respectively. For example, oxidation with a Jones reagent is exemplified.

In one embodiment of the present invention, examples of the phosphorus compound in (e) the step carried out in the presence of a stoichiometric amount of the phosphorus compound include triphenylphosphine and a triphenylphosphonium salt {(tribromomethyl) triphenylphosphonium bromide, (trichloromethyl) triphenylphosphonium chloride, and the like}.

Here, the stoichiometric amount of the phosphorus compound (triphenylphosphine or the like) means that the amount of the phosphorus compound used in the step is equimolar amount or more with respect to the amount of the long-chain polyunsaturated fatty acid or the alkyl ester thereof used.

The production method that does not comprise these steps (a), (b), (c), (d), and/or step (e) is a production method suitable for production on an industrial scale from the viewpoint of an atom economy, separation from a byproduct in post-treatment of a reaction, and/or problems of waste treatment.

Examples of the production method suitable for production on an industrial scale include a method in which synthesis can be quickly performed with a short number of steps, and/or synthesis can be performed in a large quantity with a high yield. In other aspects, examples the production method suitable for the industrial scale include a method that does not include protection and/or deprotection steps (from the viewpoint of the atom economy), a method that does not require complicated operation for removing the byproduct in post-treatment of the reaction (from the viewpoint of separation from a byproduct in post-treatment of the reaction), and/or a method that does not by-produce a highly harmful substance (from the viewpoint of problems of waste treatment).

Hereinafter, examples of the present invention will be described, but the present invention is in no way limited to these examples.

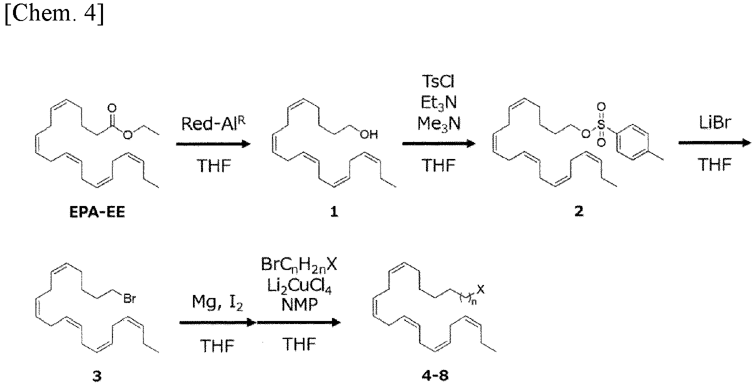

In Examples 1 to 6 presented below, the following reagents and solvents were purchased and used.

### Lithium tetrachlorocuprate (Li₂CuCl₄ solution), purchased from: Sigma-Aldrich

Super dehydrated N-methylpyrrolidone, purchased from: Wako, product code: 131-17615 Super dehydrated tetrahydrofuran, purchased from: Wako, product code: 207-17905 (containing stabilizer BHT)

### [Example 1] Preparation of ethyl (all-Z)-19,22,25,28,31-tetratriaconta pentaenoate (4)

### Step 1: Preparation of (all-Z)-5,8,11,14,17-eicosapentaen-1-ol (1)

Under a nitrogen atmosphere, super dehydrated tetrahydrofuran (300 mL) was added to ethyl (all-Z)-5,8,11,14,17-eicosapentaenoate (EPA-EE) (36.0 g, 108.9 mmol). Under ice cooling, Red-Al (trade name) (63 mL, 217.8 mmol, 2.0 eq, 70 wt% toluene solution) was gradually added dropwise. The mixture was reacted for 2 hours under ice cooling, then a mixed solution (110 mL) of ethanol:hexane = 2:3 and a 15% aqueous sodium hydroxide solution (55 mL) were added thereto, and the reaction was stopped. The mixture was extracted with hexane, and an organic phase was washed with saturated sodium chloride solution until a pH of an aqueous phase reached 9 or less. A solvent was distilled off to obtain a brown transparent oily compound 1 (32.87 g, yield: quantitative). The compound 1 was used as it was in the next step without purification.

¹H-NMR (500 MHz, CDCl₃) δ 0.97 (3H, t, J=7.5), 1.21 (1H, brs), 1.40-1.49 (2H, m), 1.55-1.64 (2H, m), 2.03-2.16 (4H, m), 2.74-2.92 (8H, m), 3.65 (2H, t, J=6.5), 5.26-5.47 (10H, m).

### Step 2: Preparation of (all-Z)-1-(4-methylbenzenesulfonyloxy)-5,8, 11,14,17-eicosapentaene (2):

Under a nitrogen atmosphere, super dehydrated tetrahydrofuran (63 mL) was added to the compound 1 (32.0 g, 110.9 mmol), and a tetrahydrofuran solution (2 mol/L) of triethylamine (46.4 mL, 332.8 mmol, 3.0 eq) and trimethylamine (5.5 mL, 11.1 mmol) was added thereto. p-Toluenesulfonyl chloride (42.3 g, 221.9 mmol, 2.0 eq) was dissolved in super dehydrated tetrahydrofuran (200 mL), and this solution was slowly added dropwise under ice cooling.

Thereafter, the reaction mixture was stirred overnight at room temperature. Water (100 mL) was added to stop the reaction. The reaction mixture was extracted with ethyl acetate, and the organic phase was washed with saturated sodium chloride solution. A solvent was distilled off to obtain a brown transparent oily compound 2 (48.97 g, yield: quantitative). The crude product was used as it was in the next step without purification.

¹H-NMR (500 MHz, CDCl₃) δ 0.96 (3H, t, J=7.5), 1.35-1.43 (2H, m), 1.61-1.70(2H, m), 1.96-2.14 (4H, m), 2.45 (3H, s), 2.76 (2H, t, J=6.6), 2.79-2.90 (6H, m), 4.03 (2H, t, J=6.5), 5.22-5.45 (10H, m), 7.34 (2H, d, J=8.0), 7.79 (2H, d, J=8.3).

In the post-treatment of this step, the byproduct is removed only by simple liquid separation operation, and complicated operation for removing the byproduct is unnecessary.

### Step 3: (all-Z)-20-bromo-3,6,9,12,15-eicosapentaene (3):

Under a nitrogen atmosphere, super dehydrated tetrahydrofuran (300 mL) was added to a mixture of the compound 2 (40.0 g, 90.36 mmol) and LiBr (15.7 g, 180.73 mmol, 2.0 eq), and the mixture was heated and refluxed at 60°C. After the reaction for 2.5 hours, the reaction mixture was left to stand to be cooled to room temperature, and water (100 mL) was added. The reaction mixture was extracted with ethyl acetate. The organic phase was washed with saturated sodium chloride solution. A solvent was distilled off to obtain a brown transparent oily crude product. Purification was performed by silica gel column chromatography (hexane) to obtain a colorless transparent oily compound 3 (25.98 g, yield: 82%).

¹H-NMR (500 MHz, CDCl₃) δ 0.97 (3H, t, J=7.5), 1.47-1.58 (2H, m), 1.81-1.93 (2H, m), 2.02-2.17 (4H, m), 2.74-2.92 (8H, m), 3.40 (2H, t, J=6.8), 5.26-5.49 (10H, m).

In the post-treatment of this step, the byproduct is removed only by simple liquid separation operation and silica gel column chromatography, and complicated operation for removing the byproduct is unnecessary.

### Step 4: ethyl (all-Z)-19,22,25,28,31-tetratriaconta pentaenoate (4):

Under a nitrogen atmosphere, super dehydrated tetrahydrofuran (13.3 mL) was added to a mixture of magnesium turnings (0.45 g, 18.63 mmol, 5.7 eq) and iodine (0.15 g, 0.58 mmol, 0.03 eq v.s.Mg), and the resulting mixture was heated and refluxed at 60°C for 30 minutes. Super dehydrated tetrahydrofuran (7.4 mL) was added to the compound 3 (1.73 g, 4.92 mmol, 1.5 eq), and the solution was added dropwise to the solution refluxing at 60°C at a flow rate of 0.9 mL/min over 10 minutes. After 30 minutes of reaction, the resultant was cooled to 0°C, and the reaction liquid was used as a Grignard reagent.

Under a nitrogen atmosphere, to ethyl 14-bromotetradecanoate (1.11 g, 3.28 mmol), super dehydrated tetrahydrofuran (14.9 mL), super dehydrated N-methylpyrrolidone (1.33 mL, 13.78 mmol, 4.2 eq), and a Li₂CuCl₄ solution (1 mL, 0.10 mmol, 0.03 eq (with respect to ethyl 14-bromotetradecanoate), 0.1 mol/L tetrahydrofuran solution) were added. Under ice cooling, a Grignard reagent (1.5 eq) was added dropwise at a flow rate of 4.3 mL/min over 5 minutes. The resultant was stirred for 30 minutes under ice cooling, and then a saturated aqueous NH₄Cl solution (20 mL) was added to stop the reaction. The mixture was extracted with ethyl acetate, and after the organic phase was washed with saturated sodium chloride solution, a solvent was distilled off to obtain a brown transparent oily crude product. Purification was performed by silica gel column chromatography (hexane and hexane:diethyl ether = 20:1) to obtain a colorless transparent oily compound 4 (1.41 g, yield: 81%).

¹H-NMR (500 MHz, CDCl₃) δ 0.98 (3H, t, J=7.5), 1.17-1.45 (31H, m), 1.56-1.68 (2H, m), 1.99-2.13 (4H, m), 2.28 (2H, t, J=7.6), 2.73-2.92 (8H, m), 4.12 (2H, q, J=7.1), 5.25-5.47 (10 H, m).

### [Example 2] One-pot synthesis of compound 3:

Under a nitrogen atmosphere, super dehydrated tetrahydrofuran (0.4 mL) was added to the compound 1 (169.5 mg, 0.59 mmol), and a tetrahydrofuran solution (2 mol/L) of triethylamine (0.25 mL, 1.82 mmol, 3.1 eq) and trimethylamine solution (0.025 mL, 0.05 mmol, 0.1 eq) was added thereto. p-Toluenesulfonyl chloride (221.2 mg, 1.16 mmol, 2.0 eq) was dissolved in super dehydrated tetrahydrofuran (1.4 mL), and the solution was slowly added dropwise under ice cooling. Thereafter, the reaction mixture was stirred at room temperature for 5 hours. Under a nitrogen atmosphere, an super dehydrated tetrahydrofuran (1.5 mL) solution of LiBr (206.9 mg, 2.38 mmol, 4.0 eq) was added to the reaction solution, and the mixture was heated and refluxed at 60°C. After the reaction for 3 hours, the reaction mixture was left to stand to be cooled to room temperature, and water (10 mL) was added. The reaction mixture was extracted with ethyl acetate, and the organic phase was washed with saturated sodium chloride solution. A solvent was distilled off to obtain a brown transparent oily crude product. Purification was performed by silica gel column chromatography (hexane) to obtain a colorless transparent oily compound 3 (177.5 mg, yield: 86%). The compound 3 obtained by the present method contains 8 wt% of (all-Z)-20-chloro-3,6,9,12,15-eicosapentane.

In this example, the compound 1 to the compound 3 could be easily produced in high yield by one-pot synthesis without isolating and purifying the compound 2. In the post-treatment of this example, the byproduct is removed only by simple liquid separation operation and silica gel column chromatography, and complicated operation for removing the byproduct is unnecessary.

### [Example 3] Preparation of (all-Z)-19,22,25,28,31-tetratriaconta pentaenoic acid (5):

Under a nitrogen atmosphere, to 14-bromotetradecanoic acid (68.8 mg, 0.22 mmol), super dehydrated tetrahydrofuran (1 mL), super dehydrated N-methylpyrrolidone (0.09 mL, 0.93 mmol, 4.2 eq), and a Li₂CuCl₄ solution (0.07 mL, 0.007 mmol, 0.03 eq (with respect to 14-bromotetradecanoic acid), 0.1 mol/L tetrahydrofuran solution) were added. Under ice cooling, a Grignard reagent (3.0 eq) prepared by a method similar to that of Example 1 (step 4) was added dropwise at a flow rate of 0.6 mL/min over 5 minutes. After stirring for 30 minutes, a saturated aqueous NH₄Cl solution (5 mL) was added to a reaction mixture to stop the reaction. The mixture was extracted with ethyl acetate, and after the organic phase was washed with saturated sodium chloride solution, a solvent was distilled off to obtain a yellow transparent oily crude product. Purification was performed by silica gel column chromatography (hexane and hexane:diethyl ether:acetic acid = 40:20:1) to obtain a white solid compound 5 (78.3 mg, yield: 70%).

¹H-NMR (500 MHz, CDCl₃) δ 0.98 (3H, t, J=7.5), 1.18-1.42 (28H, m), 1.60-1.69 (2H, m), 2.00-2.13 (4H, m), 2.35 (2H, t, J=7.5), 2.75-2.91 (8H, m), 5.25-5.47 (10H, m), 8.80 (1H, brs).

### [Example 4] Ethyl (all-Z)-13,16,19,22,25-octacosapentaenoate (6):

Under a nitrogen atmosphere, to ethyl 8-bromooctanoate (55.5 mg, 0.22 mmol), super dehydrated tetrahydrofuran (1 mL), super dehydrated N-methylpyrrolidone (0.09 mL, 0.93 mmol, 4.2 eq), and a Li₂CuCl₄ solution (0.06 mL, 0.006 mmol, 0.03 eq (with respect to ethyl 8-bromooctanoate), 0.1 mol/L tetrahydrofuran solution) were added. Under ice cooling, a Grignard reagent (3.0 eq) prepared by a method similar to that of Example 1 (step 4) was added dropwise at a flow rate of 0.3 mL/min over 10 minutes. After stirring for 1 hour, a saturated aqueous NH₄Cl solution (5 mL) was added to a reaction mixture to stop the reaction. The reaction mixture was extracted with ethyl acetate, and the organic phase was washed with saturated sodium chloride solution. A solvent was distilled off to obtain a brown transparent oily crude product. Purification was performed by silica gel column chromatography (hexane and hexane:diethyl ether = 20:1) to obtain a colorless transparent oily compound 6 (82.5 mg, yield: 84%).

¹H-NMR (500 MHz, CDCl₃) δ 0.98 (3H, t, J=7.5), 1.18-1.42 (19H, m), 1.57-1.67 (2H, m), 1.99-2.13 (4H, m), 2.28 (2H, t, J=7.6), 2.74-2.91 (8H, m), 4.12 (2H, q, J=7.1), 5.27-5.47 (10H, m).

### [Example 5] Preparation of (all-Z)-13, 16, 19,22,25-octacosapentaenoic acid (7):

Under a nitrogen atmosphere, to 8-bromooctanoic acid (50.3 mg, 0.23 mmol), super dehydrated tetrahydrofuran (1 mL) and a Li₂CuCl₄ solution (0.07 mL, 0.007 mmol, 0.03 eq (with respect to 8-bromooctanoic acid), 0.1 mol/L tetrahydrofuran solution) were added. The above solution was added dropwise at a flow rate of 0.5 mL/min over 5 minutes to a Grignard reagent (2.5 eq) prepared by a method similar to that of Example 1 (step 4) under a nitrogen atmosphere under ice cooling. After stirring for 3 hours, a saturated aqueous NH₄Cl solution (5 mL) was added to a reaction mixture to stop the reaction. The reaction mixture was extracted with ethyl acetate, and after the organic phase was washed with saturated sodium chloride solution, a solvent was distilled off to obtain a yellow transparent oily crude product. Purification was performed by silica gel column chromatography (hexane and hexane:diethyl ether:acetic acid = 40:20:1) to obtain a white solid compound 7 (40.2 mg, yield: 43%).

¹H-NMR (500 MHz, CDCl₃) δ 0.98 (3H, t, J=7.5), 1.20-1.41 (16H, m), 1.58-1.68 (2H, m), 1.95-2.15 (4H, m), 2.35 (2H, t, J=7.5), 2.72-2.91 (8H, m), 5.24-5.48 (10H, m), 10.15 (1H, brs).

### [Example 6] 2-[(all-Z)-15,18,21,24,27-triaconta pentaene-1-yl] tetrahydro-2H-pyran (8):

Under a nitrogen atmosphere, to 2-[(10-bromodecyl)oxy] tetrahydro-2H-pyran (76.4 mg, 0.24 mmol), super dehydrated tetrahydrofuran (1 mL) and a Li₂CuCl₄ solution (0.07 mL, 0.007 mmol, 0.03 eq (for 2-[(10-bromodecyl)oxy] tetrahydro-2H-pyran), 0.1 mol/L tetrahydrofuran solution) were added. The above solution was added dropwise at a flow rate of 0.3 mL/min over 5 minutes to a Grignard reagent (1.7 eq) prepared by a method similar to that of Example 1 (step 4) under a nitrogen atmosphere under ice cooling. After stirring overnight, a saturated aqueous NH₄Cl solution (5 mL) was added to a reaction mixture to stop the reaction. The reaction mixture was extracted with ethyl acetate, and after the organic phase was washed with saturated sodium chloride solution, a solvent was distilled off to obtain a brown transparent oily crude product. Purification was performed by silica gel column chromatography (hexane and toluene:hexane:diethyl ether = 90:10:1) to obtain a colorless transparent oily compound 8 (81.6 mg, yield: 67%).

¹H-NMR (500 MHz, CDCl₃) δ 0.98 (3H, t, J = 7.5), 1.19-1.41 (22H, m), 1.47-1.63 (6H, m), 1.68-1.76 (1H, m), 1.77-1.90 (1H, m), 2.00-2.12 (4H, m), 2.71-2.96 (8H, m), 3.38 (1H, dt, J₁ = 6.7, J₂ = 9.5), 3.46-3.54 (1H, m), 3.73 (1H, dt, J₁ = 7.0, J₂ = 9.5), 3.82-3.92 (1H, m), 4.58 (1H, t, J = 3.6), 5.25-5.48 (10H, m).

### Industrial Applicability

The present invention provides a method for producing a very-long-chain polyunsaturated fatty acid suitable for efficient production on an industrial scale. The present invention provides a method for producing a synthetic intermediate useful in production of a very-long-chain polyunsaturated fatty acid suitable for efficient production on an industrial scale.

## Claims

1. A method for producing a compound represented by Formula I or a salt thereof:
R¹-(CH₂)ₘ-X (I)
where R¹ is a C₁₈₋₃₀ hydrocarbon group containing from 3 to 7 double bonds;
m is an integer selected from 1 to 19;
X is CO₂H, CO₂R^{a}, or CH₂OR^{b};
R^{a} is a C₁₋₆ alkyl; and
R^{b} is an ether-forming group, a silyl ether-forming group, or an ester-forming group.

2. The method according to claim 1, comprising obtaining the compound represented by Formula I by reacting, in a solvent, a compound represented by Formula II:
R¹-MgX¹ (II)
where X¹ is a halogen atom,
with a compound represented by Formula III:
X²-(CH₂)ₘ-X (III)
where X² is selected from a halogen atom and a sulfonyloxy group.

3. The method according to claim 2, wherein the reaction is carried out in the presence of a metal catalyst.

4. The method according to claim 2 or 3, wherein the reaction is carried out in the presence of an additive.

5. The method according to any one of claims 1 to 4, wherein R¹ is a C₁₈, C₂₀ or C₂₂ hydrocarbon group containing from 3 to 6 double bonds.

6. The method according to any one of claims 1 to 5, wherein R¹ is a group represented by Formula XI:
CH₃-(CH₂-CH=CH)ₐ-(CH₂)_{b}- (XI)
where a is 5 and b is an integer selected from 2 to 14, or a is 6 and b is an integer selected from 1 to 11; or
a group represented by Formula XII:
CH₃-(CH₂)₃-(CH₂-CH=CH)_{c}-(CH₂)_{d}- (XII)
where c is 3 and d is an integer selected from 5 to 17, or c is 4 and d is an integer selected from 2 to 14.

7. The method according to claim 6, wherein R¹ is the group represented by Formula XI, in which a is 5 and b is 4, or a is 6 and b is 3, or
R¹ is the group represented by Formula XII, in which c is 3 and d is 7, or c is 4 and d is 4.

8. The method according to any one of claims 1 to 7, wherein all double bonds contained in R¹ are of a cis type.

9. The method according to any one of claims 1 to 8, wherein m is 7, 9, 11, or 13.

10. The method according to any one of claims 3 to 9, wherein the metal catalyst is selected from a copper catalyst, a nickel catalyst, a palladium catalyst, and an iron catalyst.

11. The method according to any one of claims 3 to 10, wherein the metal catalyst is selected from the group consisting of Li₂CuCl₄, CuI, CuBr, CuCl, CuBr₂, CuCl₂, CuCN, CuCl₂·2LiCl, CuCN·2LiCl, CuBr/CH₃SCH₃, Cu(acac)₂, Cu(OAc)₂, NiCl₂, Ni(acac)₂, Pd(OAc)₂, Pd(PPh₃)₄, Pd(acac)₂, PdCl₂, Fe(acac)₃, and FeCl₃.

12. The method according to any one of claims 3 to 11, wherein the metal catalyst is Li₂CuCl₄.

13. The method according to any one of claims 4 to 12, wherein the additive is selected from a nitrogen-based additive, an unsaturated hydrocarbon-based additive, and a silyl-based additive, or a combination thereof.

14. The method according to any one of claims 4 to 13, wherein the additive is selected from NMP, DMPU, HMPA, HMTA, TMEDA, 1,3-butadiene, isoprene, 1-phenylpropyne and TMS-Cl, or a combination thereof.

15. The method according to any one of claims 3 to 14, wherein an amount of the metal catalyst is from 0.1 to 20 mol% with respect to the compound represented by Formula III.

16. The method according to any one of claims 2 to 15, wherein an amount of the compound represented by Formula II is from 1 to 5 equivalents with respect to the compound represented by Formula III.

17. The method according to any one of claims 4 to 16, wherein an amount of the additive is from 0.1 to 10 equivalents with respect to the compound represented by Formula III.

18. The method according to any one of claims 2 to 17, wherein X¹ is a bromine atom.

19. The method according to any one of claims 1 to 18, wherein CH₂OR^{b} represents an ether structure having from 2 to 20 carbon atoms, a silyl ether structure having from 4 to 20 carbon atoms, or an ester structure having from 2 to 20 carbon atoms.

20. The method according to any one of claims 1 to 19, wherein R^{b} is selected from t-butyl, benzyl, p-methoxyphenylbenzyl, trityl, methoxymethyl, methoxyethoxymethyl, tetrahydropyranyl, trimethylsilyl, triethylsilyl, t-butyldimethylsilyl, t-butyldiphenylsilyl, acetyl, pivaloyl, and benzoyl.

21. The method according to any one of claims 1 to 20, wherein X is CO₂H, and the compound represented by Formula I is a very-long-chain polyunsaturated fatty acid having from 20 to 50 carbon atoms and containing from 3 to 7 double bonds, or
X is CO₂R^{a}, and the compound represented by Formula I is a C₁₋₆ alkyl ester of a very-long-chain polyunsaturated fatty acid having from 20 to 50 carbon atoms and containing from 3 to 7 double bonds.

22. The method according to any one of claims 1 to 21, wherein the compound represented by Formula I is a very-long-chain polyunsaturated fatty acid that is C₂₈₋₃₆: 5n-3, C₂₈₋₃₆: 4n-6, C₂₈₋₃₆: 6n-3, or C₂₈₋₃₆: 3n-6, or a C₁₋₆ alkyl ester thereof.

23. The method according to any one of claims 1 to 22, wherein the compound represented by Formula I is a very-long-chain polyunsaturated fatty acid that is C32: 4n-6, C34: 4n-6, C32: 5n-3, C34: 5n-3, C32: 6n-3, or C34: 6n-3, or a C₁₋₆ alkyl ester thereof.

24. The method according to any one of claims 1 to 23, wherein the compound represented by Formula I is a very-long-chain polyunsaturated fatty acid that is C32: 4n-6 or C34: 5n-3, or a C₁₋₆ alkyl ester thereof.

25. The method according to any one of claims 2 to 24, wherein the reaction comprises adding, in the solvent, a mixture containing the compound represented by Formula II:
R¹-MgX¹ (II)
to the compound represented by Formula III:
X²-(CH₂)ₘ-X (III).

26. The method according to any one of claims 4 to 25, wherein the reaction is carried out in the solvent in the presence of the metal catalyst and the additive at a temperature of from -20°C to 35°C.

27. The method according to any one of claims 4 to 26, wherein the reaction comprises adding, in the solvent, a mixture containing the compound represented by Formula II:
R¹-MgX¹ (II)
to the compound represented by Formula III:
X²-(CH₂)ₘ-X (III)
in the presence of the metal catalyst and the additive over a period of time from 5 to 80 minutes.

28. The method according to any one of claims 4 to 27, wherein the reaction comprises adding, in the solvent, a mixture containing the compound represented by Formula II:
R¹-MgX¹ (II)
to the compound represented by Formula III:
X²-(CH₂)ₘ-X (III)
in the presence of the metal catalyst and the additive at a rate of from 0.1 to 1000 mL/min.

29. The method according to any one of claims 2 to 28, further comprising converting, in the solvent, a compound represented by Formula IIc:
R¹-X¹ (IIc)
into the compound represented by Formula II:
R¹-MgX¹ (II).

30. The method according to claim 29, wherein the converting comprises adding, in the solvent, in the presence of magnesium, the compound represented by Formula IIc:
R¹-X¹ (IIc)
or a mixture containing the compound to obtain the compound represented by Formula II:
R¹-MgX¹ (II).

31. The method according to claim 30, wherein the converting comprises adding the compound represented by Formula IIc:
R¹-X¹ (IIc)
or a mixture containing the compound over 5 to 60 minutes.

32. The method according to claim 30 or 31, wherein the converting comprises adding the compound represented by Formula IIc:
R¹-X¹ (IIc)
or a mixture containing the compound at a rate of from 0.1 to 1000 mL/min.

33. The method according to any one of claims 2 to 32, further comprising:
converting a compound represented by Formula IIa:
R¹-OH (IIa)
into a compound represented by Formula IIb:
R¹-OX³ (IIb)
where X³ is a sulfonyl group;
converting the compound represented by Formula IIb into the compound represented by Formula IIc:
R¹-X¹ (IIc);
and
converting the compound represented by Formula IIc into the compound represented by Formula II:
R¹-MgX¹ (II).

34. The method according to any one of claims 1 to 19 and 21 to 32, wherein X is CH₂OR^{b}, further comprising converting a compound represented by Formula Ia:
R¹-(CH₂)ₘ-CH₂OR^{b} (Ia)
into a compound represented by Formula V:
R¹-(CH₂)ₘ-CH₂OH (V);
and
converting the compound represented by Formula V into a compound represented by Formula Ib:
R¹-(CH₂)ₘ-CO₂H (Ib),
wherein the compound represented by Formula Ib is a very-long-chain polyunsaturated fatty acid having from 20 to 50 carbon atoms and containing from 3 to 7 double bonds.

35. The method according to any one of claims 1 to 34, further comprising converting a very-long-chain polyunsaturated fatty acid into a C₁₋₆ alkyl ester.

36. Use of a compound of Formula II for preparation of a very-long-chain polyunsaturated fatty acid or a C₁₋₆ alkyl ester thereof:
R¹-MgX¹ (II)
where R¹ is a C₁₈₋₃₀ hydrocarbon group containing from 3 to 7 double bonds, and
X¹ is a halogen atom.

37. Use of a compound of Formula III for preparation of a very-long-chain polyunsaturated fatty acid or a C₁₋₆ alkyl ester thereof:
X²-(CH₂)ₘ-X (III)
where X² is selected from a halogen atom and a sulfonyloxy group;
X is CO₂H, CO₂R^{a}, or CH₂OR^{b};
R^{a} is a C₁₋₆ alkyl; and
R^{b} is an ether-forming group, a silyl ether-forming group, or an ester-forming group.

38. A method for producing a compound represented by Formula I or a salt thereof:
R¹-(CH₂)ₘ-X (I),
where R¹ is a C₁₈₋₃₀ hydrocarbon group containing from 3 to 7 double bonds,
m is an integer selected from 1 to 19;
X is CO₂H, CO₂R^{a}, or CH₂OR^{b};
R^{a} is a C₁₋₆ alkyl; and
R^{b} is an ether-forming group, a silyl ether-forming group, or an ester-forming group,
the method comprising:
(1) converting a compound represented by Formula IV:
R⁴-CO₂R² (IV)
where R⁴ is a C₁₇₋₂₉ hydrocarbon group containing from 3 to 7 double bonds and R² is a hydrogen atom or an ester-forming group,
into a compound represented by Formula IIa:
R⁴-CH₂-OH (IIa);
(2) converting the compound represented by Formula IIa into a compound represented by Formula IIb:
R⁴-CH₂-OX³ (IIb)
where X³ is a sulfonyl group;
(3) converting the compound represented by Formula IIb into a compound represented by Formula IIc:
R⁴-CH₂-X¹ (IIc)
where X¹ is a halogen atom;
(4) converting the compound represented by Formula IIc into a compound represented by Formula II:
R⁴-CH₂-MgX¹ (II);
and
(5) reacting the compound represented by Formula II with a compound represented by Formula III to convert into the compound represented by Formula I:
X²-(CH₂)ₘ-X (III)
where X² is selected from a halogen atom and a sulfonyloxy group.

39. The method according to claim 38, wherein (5) the reaction between the compound represented by Formula II and the compound represented by Formula III is performed in a solvent in the presence of a metal catalyst.

40. The method according to claim 38, wherein (1) the conversion of the compound represented by Formula IV comprises reacting the compound represented by Formula IV with a reducing agent in a solvent;
(2) the conversion of the compound represented by Formula IIa comprises reacting the compound represented by Formula IIa with a sulfonylating agent in a solvent in the presence of a base;
(3) the conversion of the compound represented by Formula IIb comprises reacting the compound represented by Formula IIb with a halogenating agent in a solvent;
(4) the conversion of the compound represented by Formula IIc comprises reacting the compound represented by Formula IIc with magnesium in a solvent; and
(5) the reaction between the compound represented by Formula II and the compound represented by Formula III comprises reacting the compound represented by Formula II with the compound represented by Formula III in a solvent in the presence of a metal catalyst.

41. The method according to any one of claims 38 to 40, wherein (2) the conversion of the compound represented by Formula IIa and (3) the conversion of the compound represented by Formula IIb are performed by a one-pot synthesis.

42. The method according to any one of claims 38 to 41, wherein the method comprises a one-pot synthesis for preparing the compound represented by Formula IIc in (3), the one-pot synthesis comprising
(2) reacting, in a solvent, the compound represented by Formula IIa with TsCl in the presence of one, or two or more amines to adjust the compound represented by Formula IIb:
R⁴-CH₂-OX³ (IIb)
where X³ is toluenesulfonyl, and
(3) reacting the compound represented by Formula IIb with LiBr without isolation to prepare the compound represented by Formula IIc:
R⁴-CH₂-X¹ (IIc)
where X¹ is a bromine atom.

43. The method according to any one of claims 38 to 42, wherein (1) the conversion of the compound represented by Formula IV is performed by reacting, in a solvent, the compound represented by Formula IV with sodium bis(2-methoxyethoxy) aluminum hydride.

44. A production method for obtaining a very-long-chain polyunsaturated fatty acid having from 20 to 50 carbon atoms and containing from 3 to 7 double bonds or a C₁₋₆ alkyl ester thereof by converting a long-chain polyunsaturated fatty acid having from 18 to 30 carbon atoms and containing from 3 to 7 double bonds or a C₁₋₆ alkyl ester thereof, the production method comprising, in the conversion step, reacting a Grignard reagent derived from the long-chain polyunsaturated fatty acid or the C₁₋₆ alkyl ester thereof with a ω-halogenated C₂₋₂₀ fatty acid or a C₁₋₆ alkyl ester thereof to obtain the very-long-chain polyunsaturated fatty acid or the C₁₋₆ alkyl ester thereof,
wherein in the conversion step, one or more steps selected from (a) to (e) are not comprised: (a) protecting a compound having a hydroxy group into a compound having a protected hydroxy group; (b) deprotecting a compound having a protected hydroxy group into a compound having a hydroxy group; (c) oxidizing an alcohol to an aldehyde or a carboxylic acid; (d) oxidizing an aldehyde to a carboxylic acid; and (e) a step to be performed on a compound derived from the long-chain polyunsaturated fatty acid or the C₁₋₆ alkyl ester thereof in the presence of a stoichiometric amount of a phosphorus compound.

45. A production method for obtaining a very-long-chain polyunsaturated fatty acid having from 20 to 50 carbon atoms and containing from 3 to 7 double bonds or a _{C1-6} alkyl ester thereof by converting a long-chain polyunsaturated fatty acid having from 18 to 30 carbon atoms and containing from 3 to 7 double bonds or a C₁₋₆ alkyl ester thereof,
the production method comprising, in the conversion step, reacting a Grignard reagent derived from the long-chain polyunsaturated fatty acid or the C₁₋₆ alkyl ester thereof with a ω-halogenated C₂₋₂₀ fatty acid or a C₁₋₆ alkyl ester thereof in the presence of a metal catalyst to obtain the very-long-chain polyunsaturated fatty acid or the C₁₋₆ alkyl ester thereof, wherein in the conversion step, one or more steps selected from (a) to (e) are not comprised: (a) protecting a compound having a hydroxy group into a compound having: a protected hydroxy group forming a C₁₋₂₀ ether structure; a protected hydroxy group forming a C₃₋₂₀ silyl ether structure; or a protected hydroxy group forming a C₁₋₂₀ ester structure; (b) deprotecting a compound having: a protected hydroxy group forming a C₁₋₂₀ ether structure; a protected hydroxy group forming a C₃₋₂₀ silyl ether structure; or a protected hydroxy group forming a C₁₋₂₀ ester structure into a compound having a hydroxy group; (c) oxidizing a compound having a hydroxymethyl group to a compound having a formyl group or a carboxy group; (d) oxidizing a compound having a formyl group to a compound having a carboxy group; and (e) a step to be performed on a compound derived from the long-chain polyunsaturated fatty acid or a C₁₋₆ alkyl ester thereof in the presence of a stoichiometric amount of triphenylphosphine or triphenylphosphonium salt.
